(19) Europäisches Patentamt
European Patent Office
Office européen des brevets

(11) **EP 4 628 041 A1**

(12) **EUROPEAN PATENT APPLICATION**

(43) Date of publication:
**08.10.2025 Bulletin 2025/41**

(51) International Patent Classification (IPC):
**A61B 34/30** $^{(2016.01)}$  **A61B 90/00** $^{(2016.01)}$

(21) Application number: **25165931.4**

(22) Date of filing: **25.03.2025**

(52) Cooperative Patent Classification (CPC):
**A61B 34/30;** A61B 2090/064; A61B 2090/067

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.03.2024 GB 202404322**

(71) Applicant: **CMR Surgical Limited
Cambridge CB24 9NG (GB)**

(72) Inventors:
• **WEBSTER-SMITH, David William Haydn
  Cambridge, CB24 9NG (GB)**
• **TODD, Robin Elizabeth McKenzie
  Cambridge, CB24 9NG (GB)**
• **ANDERSON, Richard John Dormer
  Cambridge, CB24 9NG (GB)**
• **ASCAH-COALLIER, Isabelle
  Cambridge, CB24 9NG (GB)**

(74) Representative: **Slingsby Partners LLP
1 Kingsway
London WC2B 6AN (GB)**

(54) **CONTROL SYSTEM FOR CONTROLLING A SURGICAL ROBOT ARM**

(57) A control system for controlling a surgical robot arm, the surgical robot arm having a drive mechanism configured for driving a robotic surgical instrument, said robotic surgical instrument comprising a first end effector element and a second end effector element, the drive mechanism comprising one or more interface elements configured to interface with the robotic surgical instrument for controlling a spread angle between the first end effector element and the second end effector element, the control system being configured to: cause an instrument drive force applied at the one or more interface elements to be varied; measure, at each of a plurality of times, the instrument drive force applied at the one or more interface elements; determine, for each of the plurality of times, a force behaviour value using a derivative of the measured instrument drive force with respect to the time at that time; determine an offset value in dependence on the plurality of determined force behaviour values; and control, in dependence on the offset value, the surgical robot arm to control the spread angle between the first end effector element and the second end effector element of the robotic surgical instrument.

FIG. 18

EP 4 628 041 A1

# EP 4 628 041 A1

**Description**

FIELD

This invention relates to a control system for controlling a surgical robot arm.

BACKGROUND

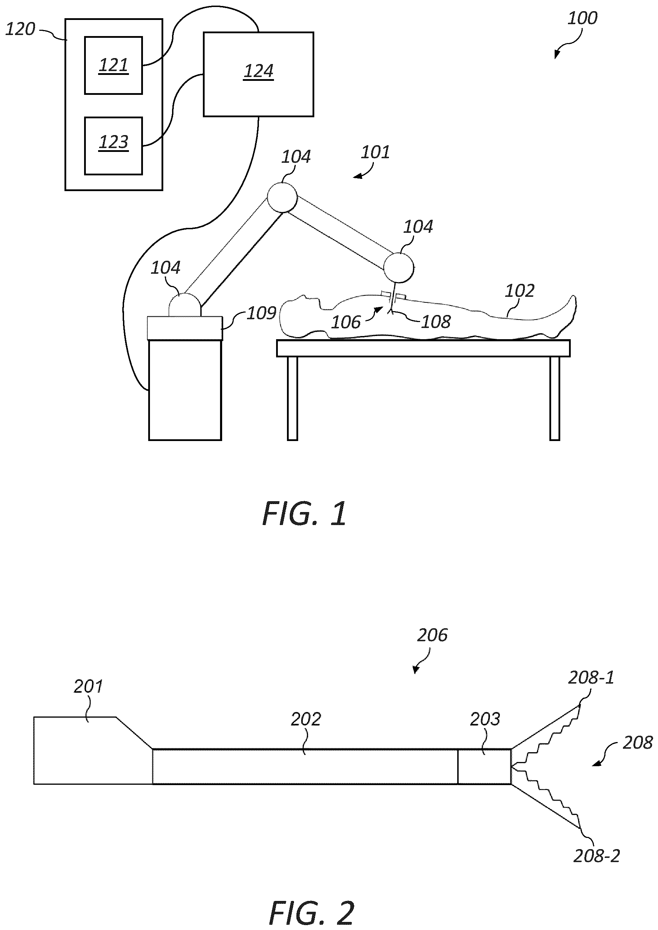

It is known to use robots for assisting and performing surgery. Figure 1 illustrates an example surgical robotic system 100, which comprises a surgical robot arm 101 for manipulating tissue. The surgical robot arm 101 comprises a base 109. The base supports the surgical robot arm, and is itself attached rigidly to, for example, the operating theatre floor, the operating theatre ceiling or a trolley. The surgical robot arm 101 is articulated by means of multiple joints 104 along its length, which are used to locate a robotic surgical instrument 106 in a desired location relative to the patient 102. The robotic surgical instrument 106 could, for example, be a cutting or grasping device. A robotic surgical instrument 106 is attached to the distal end of the surgical robot arm 101. The robotic surgical instrument 106 is positioned within the body of the patient 102 so as to access a surgical site. At its distal end the robotic surgical instrument comprises an end effector 108 for performing aspects of a medical procedure. This type of medical procedure is often referred to as a minimally invasive surgical procedure.

The configuration of the surgical robot arm 101, and the configuration of the robotic surgical instrument 106 driven by the surgical robot arm 101, may be remotely controlled in response to inputs received at a remote surgeon console 120. A surgeon may provide inputs to the remote console. The remote surgeon console may comprise one or more surgeon input devices 123. For example, these may take the form of one or more hand controllers and/or foot pedals. A video feed of the surgical site may be captured by an endoscope, often attached to a further surgical robot arm (not shown in Figure 1 for simplicity), and displayed at a display 121 of the remote surgeon console.

A control system 124 connects the surgeon console 120 to the surgical robot arm 101. The control system 124 receives inputs from the surgeon input device(s) 123 and converts those inputs to control signals for controlling the surgical robot arm 101, and the robotic surgical instrument 106 driven by the surgical robot arm 101. It is important that the surgeon feels (e.g. perceives) that the movements of the surgical robot arm 101 and the robotic surgical instrument 106 accurately reflect the inputs they provide to the surgeon input device(s) 123.

SUMMARY OF THE INVENTION

According to aspects of the invention, there are provided control systems for controlling a surgical robot arm as set out in the accompanying claims.

According to other aspects of the invention, there are provided surgical robotic systems as set out in the accompanying claims.

According to other aspects of the invention, there are provided methods of controlling a surgical robot arm as set out in the accompanying claims.

BRIEF DESCRIPTION OF THE DRAWINGS

The present invention will now be described by way of example with reference to the accompanying drawings. In the drawings:

Figure 1 illustrates a surgical robotic system.
Figure 2 illustrates a robotic surgical instrument.
Figures 3 and 4 illustrate the distal end of a robotic surgical instrument.
Figure 5 illustrates a robotic surgical instrument.
Figure 6A illustrates a drive mechanism for driving a robotic surgical instrument.
Figure 6B illustrates a robotic surgical instrument attached to a drive mechanism for driving that robotic surgical instrument.
Figure 7 illustrates a spread angle between first and second end effector elements of a robotic surgical instrument.
Figure 8 illustrates a first experimental plot of applied instrument drive force against predicted resultant spread angle.
Figure 9 illustrates a first set of steps performed by the control system for controlling a robotic surgical instrument in dependence on a determined spread offset value.
Figure 10 illustrates a first set of steps performed by the control system for controlling a robotic surgical instrument in dependence on a determined interface position offset value.
Figure 11 illustrates a second experimental plot of applied instrument drive force against predicted resultant spread angle.

Figure 12 illustrates an experimental plot of the first derivative of applied instrument drive force with respect to predicted resultant spread angle against resultant spread angle.

Figure 13 illustrates a second set of steps performed by the control system for controlling a robotic surgical instrument in dependence on a determined spread offset value.

Figure 14 illustrates a second set of steps performed by the control system for controlling a robotic surgical instrument in dependence on a determined interface position offset value.

Figure 15A illustrates the true zero configuration and spread offset value relative to predicted resultant spread angles when closing a robotic surgical instrument.

Figure 15B illustrates the true zero configuration and spread offset value relative to lost motion compensating predicted resultant spread angles when closing a robotic surgical instrument.

Figure 16 illustrates a first set of steps performed by the control system for compensating for lost motion.

Figure 17 illustrates a set of steps performed by the control system for determining a calibrated spread angle.

Figure 18 illustrates experimental plots of measured instrument drive force, first derivative of measured instrument drive force with respect to time, and predicted resultant spread angle against time.

Figure 19 illustrates a set of steps that can be performed by a control system for controlling a surgical robot arm in dependence on a determined offset value.

Figure 20 illustrates a second set of steps performed by the control system for controlling a robotic surgical instrument in dependence on an approximated lost motion compensation value.

Figure 21 illustrates a first example lost motion compensation function.

Figures 22A and 22B illustrate example uses of the first example lost motion compensation function to approximate lost motion compensation values.

Figure 23 illustrates a second example lost motion compensation function.

## DETAILED DESCRIPTION OF THE DRAWINGS

The following description is presented to enable any person skilled in the art to make and use the invention, and is provided in the context of a particular application. Various modifications to the disclosed embodiments will be readily apparent to those skilled in the art.

The general principles defined herein may be applied to other embodiments and applications without departing from the spirit and scope of the present invention. Thus, the present invention is not intended to be limited to the embodiments shown, but is to be accorded the widest scope consistent with the principles and features disclosed herein.

As described herein with reference to Figure 1, in surgical robotic system 100, a control system 124 connects a surgeon console 120 to the surgical robot arm 101. The control system receives inputs from the surgeon input device(s) 123 and converts those inputs to control signals for controlling the surgical robot arm 101, and the robotic surgical instrument 106 driven by the surgical robot arm 101. The control system 124 sends those control signals to the surgical robot arm 101, which drives the surgical instrument 106 accordingly. The control system may periodically (e.g. at irregular or at predetermined time intervals) receive inputs the surgeon input device(s) 123, and periodically (e.g. at irregularly or at predetermined time intervals) send corresponding control signals to the surgical robot arm 101. That is, the surgical robotic system 100 comprises a control system 124 for controlling a surgical robot arm 101.

The control system 124 comprises a processor and a memory. The memory stores, in a non-transient way, software code that can be executed by the processor to cause the processor to control the surgical robot arm 101 in the manner described herein.

The control system 124 may be separate from the remote surgeon console 120 and the surgical robot arm 101. The control system 124 may be collocated with the remote surgeon console 120. The control system 124 may be collocated with the surgical robot arm 101. The control system 124 may be distributed between the remote surgeon console 120 and the surgical robot arm 101.

Figure 2 illustrates a typical robotic surgical instrument 206 for use in a minimally invasive surgical procedure. When attached thereto, the robotic surgical instrument 206 can be driven by a surgical robot arm (e.g. surgical robot arm 101 shown in Figure 1). The robotic surgical instrument 206 comprises a proximal base 201 by means of which the robotic surgical instrument can connect to (e.g. attach to) a surgical robot arm (e.g. surgical robot arm 101 shown in Figure 1). A shaft 202 extends between base 201 and an articulation 203. Articulation 203 connects the shaft 202 to an end effector 208. The end effector 208 is at the distal end of the robotic surgical instrument 206. The end effector 208 comprises a first end effector element 208-1 and a second end effector element 208-2. In Figure 2, a pair of serrated jaws are illustrated as the first and second end effector elements 208-1, 208-2. The articulation 203 permits the end effector 208, and one or both of its end effector elements 208-1, 208-2, to move relative to the shaft 202. It is desirable - although not essential - for at least two degrees of freedom (e.g. pitch and yaw) to be provided to the motion of the end effector 208 by means of the articulation.

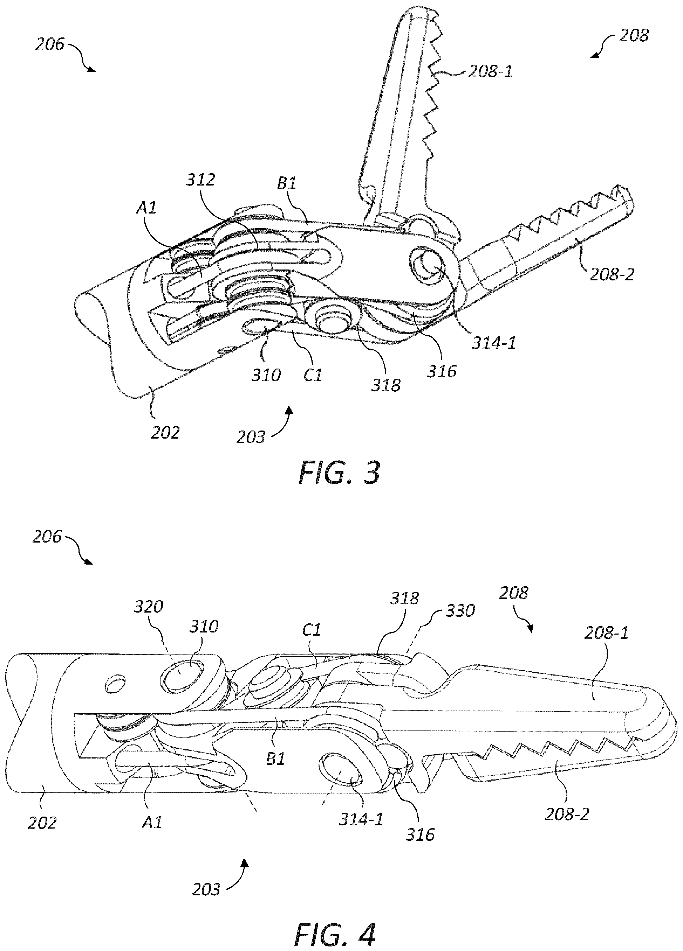

Figures 3 to 7 illustrate one specific example of a robotic surgical instrument and a drive mechanism configured for driving that robotic surgical instrument. It is to be understood that the robotic surgical instrument and the drive mechanism shown in Figures 3 to 7 are described by way of example only for the purpose of aiding the reader's understanding of the principles described herein. It is to be understood that the principles described herein could be applied to various different robotic surgical instruments and/or various different drive mechanisms. For example, in Figures 3 to 7, the robotic surgical instrument uses a drive transfer mechanism comprising cables and pulleys, that drive mechanism being capable of independently controlling the first and second end effector elements. In an alternative example to which the principles described herein could also be applied, the robotic surgical instrument may use a drive transfer mechanism comprising one or more push-rods and a rack and pinion mechanism. In alternative examples to which the principles described herein could also be applied, the robotic surgical instrument may use a drive mechanism that is capable of: independently controlling the first and second end effector elements; controlling only one of the end effector elements; or controlling both the first and second end effector elements in tandem. The skilled person would be aware of numerous suitable robotic surgical instruments and drive mechanisms to which the principles described herein could be applied.

Figures 3 and 4 illustrate the distal end of a robotic surgical instrument 206. At its distal end the robotic surgical instrument 206 comprises an end effector 208 for performing aspects of a medical procedure. The end effector 208 comprises a first end effector element 208-1 and a second end effector element 208-2. Figure 3 shows the robotic surgical instrument 206 in an open configuration. In an open configuration, the inner surfaces of the first end effector element 208-1 and the second end effector element 208-2 are not in contact. In other words, in an open configuration, an inner surface of the first end effector element 208-1 is not exerting a force against an inner surface of the second end effector element 208-2, and vice versa. Figure 4 shows the robotic surgical instrument 206 in a closed configuration. In a closed configuration, the inner surfaces of the first end effector element 208-1 and the second end effector element 208-2 are in contact. In other words, in a closed configuration, an inner surface of the first end effector element 208-1 may be exerting a force against an inner surface of the second end effector element 208-2, and vice versa.

The end effector 208 is permitted to move relative to shaft 202 by means of articulation 203 comprising two yaw joints 314-1 and 314-2 and, optionally, a pitch joint 310. Optional pitch joint 310 enables the end effector 208 as a whole to rotate about a pitch axis 320. Yaw joints 314-1 and 314-2 enable the end effector elements 208-1 and 208-2, respectively, to rotate about a yaw axis 330. That is, the first and second end effector elements 208-1 and 208-2 are rotatably mounted with respect to one another. In the perspective views shown in Figures 3 and 4, only one of the two yaw joints is visible - yaw joint 314-1, which permits end effector element 208-1 to rotate about the yaw axis 330. As will be described in further detail herein, the first and second end effector elements 208-1 and 208-2 can be caused to "spread" by causing one or both of those end effector elements 208-1, 208-2 to rotate away from the other about the yaw axis 330. As such, the angle between the first end effector element 208-1 and the second end effector element 208-2 caused by the yawing of those end effector elements can be termed the spread angle.

In the robotic surgical instrument 206 shown in Figures 3 and 4, end effector elements 208-1 and 208-2 rotate about a common yaw axis 330 - although it is to be understood that this need not be the case. For example, the first and second end effector elements 208-1 and 208-2 may rotate about different yaw axes - e.g. yaw axes that are offset with respect to one another.

In the robotic surgical instrument 206 shown in Figures 3 and 4, the first and second end effector elements 208-1 and 208-2 can rotate independently about the yaw axis 330. For example, one end effector element may rotate whilst the other end effector element remains stationary, and vice versa. It is to be understood that this need not be the case. For example, the first and second end effector elements 208-1 and 208-2 may not be independently rotatable - e.g. the first and second end effector elements 208-1 and 208-2 may only be rotatable about the yaw axis in opposing directions in tandem. In other examples, only one of the end effector elements may be rotatable, whilst the other end effector element remains stationary (e.g. fixed in place relative to the articulation 203).

In the robotic surgical instrument 206 shown in Figures 3 and 4, joints 310, 314 and 316 are driven by a drive transfer mechanism comprising cables and pulleys. In this example: rotation of the end effector 208 about pitch joint 310 is driven by cable A1 causing the rotation of pitch pulley 312; rotation of the first end effector element 208-1 about the first yaw joint 314-1 is driven by cable B1 causing the rotation of first yaw pulley 316; and rotation of the second end effector element 208-2 about the second yaw joint 314-2 is driven by cable C1 causing the rotation of second yaw pulley 318. It is to be understood that Figures 3 and 4 shows just one example of a cable and pulley drive transfer mechanism, and that the various other suitable drive transfer mechanisms could be used to permit the end effector 208 to move relative to the shaft 202.

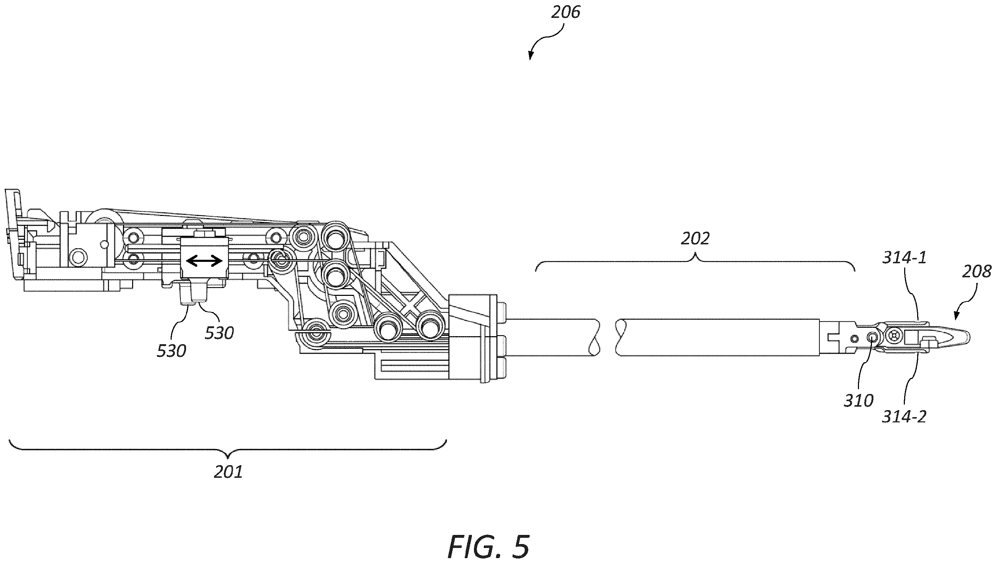

Figure 5 illustrates the proximal end of robotic surgical instrument 206 in further detail. As described herein, at its proximal end the robotic surgical instrument 206 comprises a base 201 by means of which the robotic surgical instrument can connect to (e.g. attach to) a surgical robot arm (e.g. surgical robot arm 101 shown in Figure 1). In the base 201, the drive transfer mechanism of robotic surgical instrument 206 comprises three instrument interface elements 530 (although only two are visible in Figure 5). Each instrument interface element 530 is secured to a respective cable (e.g. cables A1, B1 and C1 shown in Figures 3 and 4). That cable extends, via a series of pulleys, down shaft 202 for driving (e.g. transferring a driving force to) a respective joint of the robotic surgical instrument 206 - e.g. a respective one of pitch joint 310 and yaw joints 314-1 and 314-2 as described herein. Via said drive transfer mechanism, linear movement of an instrument interface element 530 (illustrated in Figure 5 using a double headed arrow) causes rotation about a respective joint 310, 314-1, 314-2 of the robotic surgical instrument 206. The instrument interface elements 530 are exposed so as to be capable of interfacing with a drive mechanism of the surgical robot arm (e.g. surgical robot arm 101 shown in Figure 1). It is to be understood that Figure 5 shows just one example of a cable and pulley drive transfer mechanism, and that the various other suitable drive transfer mechanisms (e.g. other arrangements using cables to transfer drive forces, or alternative arrangements using pushrods to transfer drive forces) could be used to permit the end effector 208 to move relative to the shaft 202.

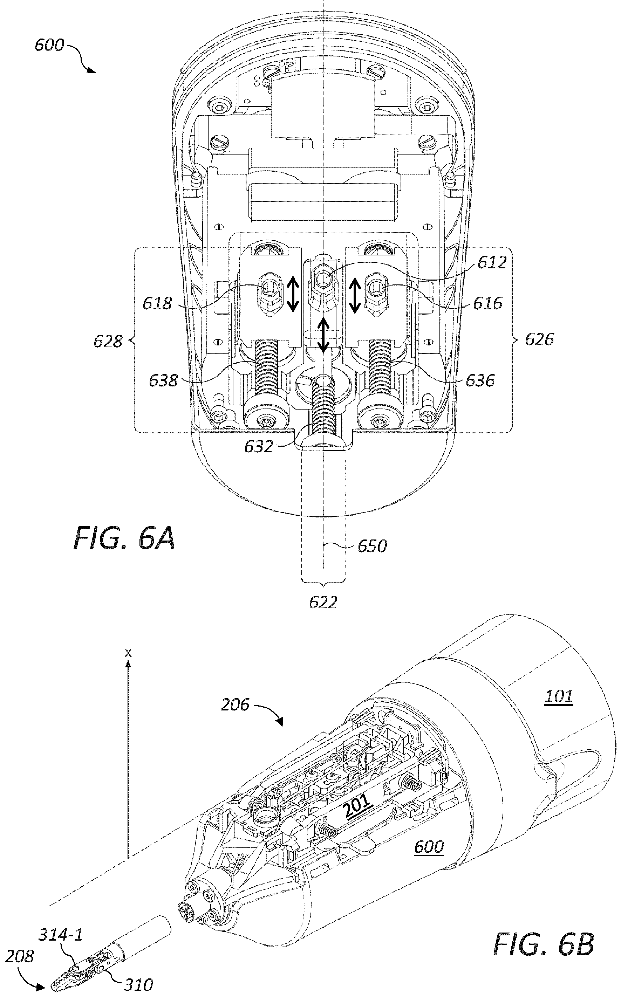

At its distal end, the surgical robot arm (e.g. surgical robot arm 101 shown in Figure 1) comprises a drive mechanism configured for driving a robotic surgical instrument. Figure 6A illustrates an example drive mechanism 600 configured for driving robotic surgical instrument 206. The drive mechanism 600 comprises three drive assemblies 622, 626, 628. In the example shown in Figure 6A: the first drive assembly 622 is configured for driving rotation of the end effector 208 about pitch joint 310; the second drive assembly 626 is configured for driving rotation of the first end effector element 208-1 about the first yaw joint 314-1; and the third drive assembly 628 is configured for driving rotation of the second end effector element 208-2 about the second yaw joint 314-2.

Each drive assembly comprises a respective interface element 612, 616, 618 driven by a respective lead screw 632, 636, 638. The lead screws 632, 636, 638 are driven to rotate in order to convey linear motion (as illustrated in Figure 6 using double headed arrows) to the interface elements 612, 616, 618. In other words, the interface elements 612, 616, 618 are each driveable along a direction parallel to the longitudinal axis 650 of the distal end of the surgical robot arm along their respective lead screw 632, 636, 638.

Interface elements 612, 616, 618 each have a complimentary shape to their respective instrument interface element 530 (shown in Figure 5). For example, the interface elements 612, 616, 618 may be shaped so as to receive their respective instrument interface element 530, or vice versa. As such, each interface element 612, 616, 618 can apply a driving force to, and thereby cause linear movement of, its respective instrument interface element 530.

Each of the drive assemblies 622, 626, 628 comprises a force sensor (not shown in Figure 6A) for sensing the force applied at its respective interface element 612, 616, 618. For example, each force sensor may sense the force applied to an interface element - e.g. by its lead screw. Alternatively, or additionally, each force sensor may sense the force applied by an interface element - e.g. to its respective instrument interface element 530. In theory, the force applied to an interface element should equal the force applied by that interface element - although in reality some losses may be experienced, e.g. owing to friction in the drive mechanism. The sensed forces are output to the control system 124.

Each of the drive assemblies 622, 626, 628 comprises a position sensor (not shown in Figure 6A) for measuring the position of its respective interface element 612, 616, 618. In examples, the measured position may be: a position (e.g. a linear position) of the interface element along its direction of travel; or an angular orientation of a lead screw that is indicative of the position of the interface element that it drives. The position may be defined relative to a neutral (e.g. resting or "zero") position of the interface element, or may be defined relative to one end of its possible travel. The position of an interface element may be defined in absolute units (e.g. millimetres), or as a proportion (e.g. fraction or percentage) of the total possible travel of that interface element. For example, the position of an interface element may be defined as plus or minus X millimetres relative to a neutral (e.g. resting or "zero") position. The neutral (e.g. resting or "zero") position may be a position midway (e.g. halfway, although not necessarily) along the travel of that interface element. The position of an interface element may be defined as being "positive" (e.g. "plus" X millimetres) when the interface element is in a more distal position than its neutral (e.g. resting or "zero") position. Alternatively, the position of an interface element may be defined as being "positive" (e.g. "plus" X millimetres) when the interface element is in a more proximal position than its neutral (e.g. resting or "zero") position. The measured positions are output to the control system 124.

Figure 6B illustrates robotic surgical instrument 206 attached to drive mechanism 600. During attachment, the base 201 of the surgical instrument 206 is lowered into the drive mechanism 600 causing the instrument interface elements 530 of surgical instrument 206 to engage with their respective interface elements 612, 616, 618 of drive mechanism 600. The instrument interface elements 530 may engage directly with their respective interface elements 612, 616, 618. Alternatively, the instrument interface elements 530 may be separated from their respective interface elements 612, 616, 618 by an intermediate component (e.g. a sterile barrier - such as a sheet of flexible material) so as to engage indirectly with their respective interface element 612, 616, 618. In this way, the interface elements 612, 616, 618 of drive mechanism 600 can each apply an instrument drive force to their respective instrument interface element 530 of the drive transfer mechanism of robotic surgical instrument 206. The drive transfer mechanism of robotic surgical instrument 206 is configured for transferring those forces to the articulation 203 of robotic surgical instrument 206 - e.g. comprising the pitch and yaw joints 310, 314-1 and 314-2 as described herein. As such, the drive transfer mechanism of robotic surgical instrument 206 is configured for transferring driving forces from interface elements 616 and 618 to the first end effector element 208-1 and the second end effector element 208-2, respectively. When the robotic surgical instrument 206 is attached to drive mechanism 600, the surgical robot arm 101 can drive movement of the first and second end effector elements 208-1, 208-2 by driving movement of the interface elements 612, 616, 618.

In the example robotic surgical instrument and drive mechanism shown in Figures 5 to 6, interface elements 616 and 618 are configured to interface with the robotic surgical instrument 206 for controlling the spread angle between the first end effector element 208-1 and the second end effector element 208-2. It is to be understood that, in alternative examples where the first and second end effector elements of a robotic surgical instrument are not independently rotatable (e.g. yawing of both the first and second end effector elements is driven in tandem by a common pulley, gear, or rack and pinion mechanism), or where only one of the end effector elements is rotatable whilst the other end effector element remains stationary (e.g. fixed in place relative to the articulation), the drive mechanism need only comprise one interface element for driving the yaw rotation of one or both of the first and second end effector elements so as to control the spread angle between those end effector elements. The skilled person would be aware of numerous suitable robotic surgical instruments and drive mechanisms of this alternative type. As such, according to the principles described herein, the drive mechanism comprises one or more interface elements configured to interface with the robotic surgical instrument for controlling a spread angle between the first end effector element and the second end effector element.

The control system 124 may use kinematic equations to relate the measured position of the one or more interface elements of the drive mechanism to a resultant spread angle between the first end effector element and the second end effector element. These kinematic equations may be dependent on the geometry of one or more components (e.g. pulleys, gears, rack and pinion mechanism etc.) of the drive transfer mechanism of the robotic surgical instrument.

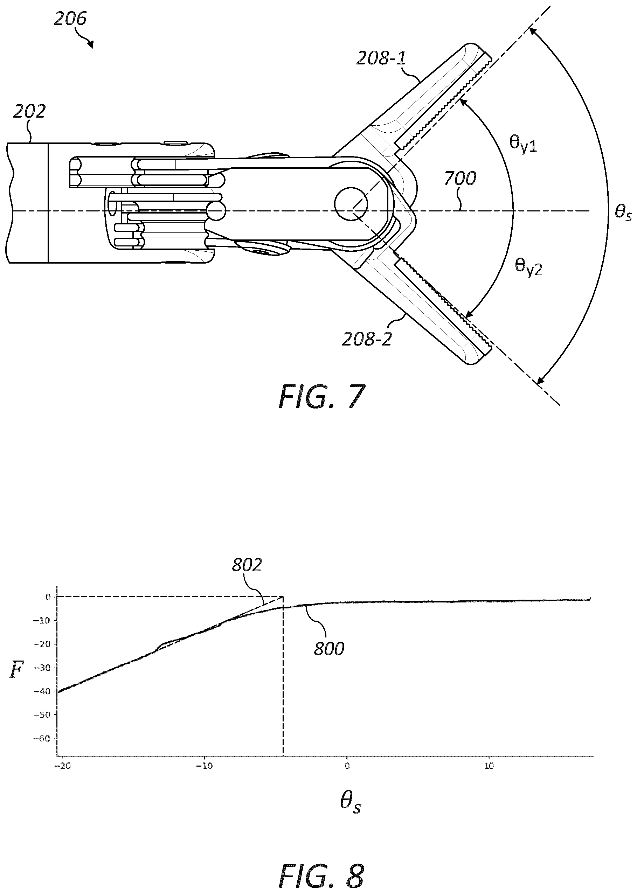

An example set of kinematic equations suitable for use with the example robotic surgical instrument and drive mechanism shown in Figures 3 to 6 is described herein with reference to Figure 7 - which illustrates a spread angle $\theta_s$ between first and second end effector elements of a robotic surgical instrument. Figure 7 illustrates the distal end of robotic surgical instrument 206 comprising first end effector element 208-1 and second end effector element 208-2. Figure 7 shows the robotic surgical instrument 206 in an open configuration.

In Figure 7, the first end effector element 208-1 is rotated about its yaw joint by a yaw angle $\theta_{y1}$. The yaw angle $\theta_{y1}$ may be defined relative to the longitudinal axis 700 of shaft 202. The yaw angle $\theta_{y1}$ shown in Figure 7, in which the first end effector element 208-1 is "above" the longitudinal axis 700 (as viewed in Figure 7), may be defined as a positive yaw angle. Were the first end effector element 208-1 to rotate to a position "below" the longitudinal axis 700 (as viewed in Figure 7), its yaw angle may then be defined as a negative yaw angle. The yaw angle $\theta_{y1}$ of the first end effector element 208-1 can be predicted using Equation 1, in which $q_{11}$ is the measured position of the interface element 616, $r_{y1}$ is the radius of the first yaw pulley 316, $q_{10}$ is the measured position of the interface element 612, $r_p$ is the radius of the pitch pulley 312, and $\lambda$ is a pitch correction factor. By way of example only, the radius $r_{y1}$ of the first yaw pulley 316 may be 2.1 mm, the radius $r_p$ of the pitch pulley 312 may be 2.0mm, and the pitch correction factor $\lambda$ may be 1.75 mm/rad. Suitable values for these parameters may be stored by the control system 124 for each robotic surgical instrument and/or each robotic surgical instrument may store its parameters in a memory local to that instrument such that those parameters can be provided to the control system 124 when that instrument is attached to the surgical robot arm.

$$\theta_{y1} = \frac{q_{11}}{r_{y1}} - \frac{\lambda q_{10}}{r_p r_{y1}} \qquad (1)$$

In Figure 7, the second end effector element 208-2 is rotated about its yaw joint by a yaw angle $\theta_{y2}$. The yaw angle $\theta_{y2}$ may be defined relative to the longitudinal axis 700 of shaft 202. The yaw angle $\theta_{y2}$ shown in Figure 7, in which the second end effector element 208-2 is "below" the longitudinal axis 700 (as viewed in Figure 7), may be defined as a negative yaw angle. Were the second end effector element 208-2 to rotate to a position "above" the longitudinal axis 700 (as viewed in Figure 7), its yaw angle may then be defined as a positive yaw angle. The yaw angle $\theta_{y2}$ of the second end effector element 208-2 can be predicted using Equation 2, in which $q_9$ is the measured position of the interface element 618, $r_{y2}$ is the radius of the second yaw pulley 318, $q_{10}$ is the measured position of the interface element 612, $r_p$ is the radius of the pitch pulley 312, and $\lambda$ is a pitch correction factor. By way of example only, the radius $r_{y2}$ of the second yaw pulley 318 may be 2.1mm, the radius $r_p$ of the pitch pulley 312 may be 2.0mm, and the pitch correction factor $\lambda$ may be 1.75 mm/rad. Suitable values for these parameters may be stored by the control system 124 for each robotic surgical instrument and/or each robotic surgical instrument may store its parameters in a memory local to that instrument such that those parameters can be provided to the control system 124 when that instrument is attached to the surgical robot arm.

$$\theta_{y2} = -\frac{q_9}{r_{y2}} - \frac{\lambda q_{10}}{r_p r_{y2}} \qquad (2)$$

The spread angle between the first end effector element 208-1 and the second end effector element 208-2 can be predicted using Equation 3.

$$\theta_s = (\theta_{y1} - \theta_{y2}) \qquad (3)$$

Written out in full, by incorporating the relationships defined by Equations 1 and 2 into Equation 3, the spread angle between the first end effector element 208-1 and the second end effector element 208-2 can be predicted using Equation 4.

$$\theta_s = \left(\frac{q_{11}}{r_{y1}} - \frac{\lambda q_{10}}{r_p r_{y1}}\right) - \left(-\frac{q_9}{r_{y2}} - \frac{\lambda q_{10}}{r_p r_{y2}}\right) \qquad (4)$$

It follows that, in examples where the radius $r_{y1}$ of the first yaw pulley 316 is equal to the radius $r_{y2}$ of the second yaw pulley 318, the "pitch" terms in Equation 4 cancel, such that the spread angle between the first end effector element 208-1 and the second end effector element 208-2 can be predicted using Equation 5.

$$\theta_s = \frac{q_{11}}{r_{y1}} + \frac{q_9}{r_{y2}} \qquad (5)$$

It is noted that the kinematic equations defined in Equations 1 to 5 are specific examples suitable for the example robotic surgical instrument and drive mechanism shown in Figures 3 to 6. It is to be understood that the skilled person would have no difficulty generating suitable kinematic equations for other suitable robotic surgical instruments and drive mechanisms.

As described herein, it is important that the surgeon feels (e.g. perceives) that the movements of the robotic surgical instrument 106 accurately reflect the inputs they provide to the surgeon input device(s) 123.

The kinematic equations described above rely on a number of assumptions. For example, the kinematic equations assume that the drive transfer mechanism of the robotic surgical instrument is infinitely stiff and/or experiences negligible friction, such that no motion is lost (e.g. to friction or cable stretch) between the interface elements 616, 618 of the drive mechanism 600 and the yaw joints 314-1 and 314-2 of the robotic surgical instrument 206. In addition, the kinematic equations do not account for manufacturing variability in the drive mechanism and/or the drive transfer mechanism of the robotic surgical instrument. Nor do the kinematic equations account for the variability introduced by positioning of a sterile interface between the drive mechanism and the robotic surgical instrument. These factors may mean that the actual spread angle differs from the spread angle predicted by the control system using those kinematic equations.

For example, it has been observed by the inventors that, for certain robotic surgical instruments, the actual spread angle can be 15° or more when the kinematic equations predict spread angles of 0°. This level of error may be perceptible by the surgeon - e.g. as the robotic surgical instrument may be in an open configuration when the surgeon is commanding a closed configuration using the surgeon input device(s) 123. For other robotic surgical instruments, the inventors have observed that their end effectors can be in closed or "over-closed" configurations when the kinematic equations predict spread angles greater than 0°. In "over-closed" configurations, an inner surface of the first end effector element 208-1 is exerting a force against an inner surface of the second end effector element 208-2. This can be problematic as the surgeon may be exerting more force on an object held between the end effector elements than they were intending to. Further, during an instrument change, members of the operating room staff may find it difficult to detach a robotic surgical instrument that is in an over-closed configuration from the drive mechanism.

In the following, sets of steps that can be performed by the control system 124 for controlling the surgical robot arm 101 are described. A control system configured to perform one or more of these sets of steps can address one or more of the problems described above.

*Concept 1 - spread offset value*

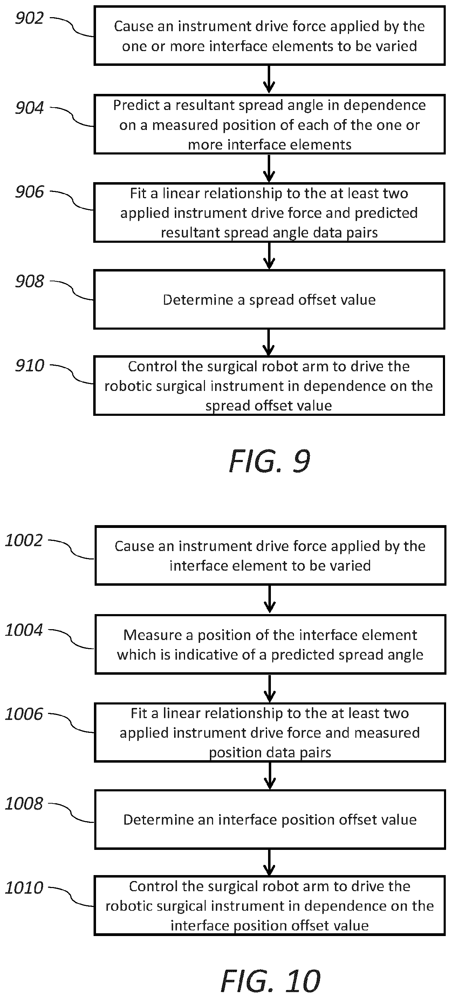

Figure 9 illustrates a first set of steps that can be performed by a control system (e.g. control system 124 shown in Figure 1) for controlling a surgical robot arm (e.g. surgical robot arm 101 shown in Figure 1) in dependence on a determined spread offset value. The surgical robot arm has a drive mechanism configured for driving a robotic surgical instrument. The robotic surgical instrument comprises a first end effector element and a second end effector element. The drive mechanism comprises one or more interface elements configured to interface with the robotic surgical instrument for controlling a spread angle between the first end effector element and the second end effector element.

In step 902, the control system is configured to cause an instrument drive force applied at the one or more interface elements to be varied. The applied forces may be measured using force sensors - such as those described with reference to Figure 5. The control system is configured to perform step 902 whilst an inner surface of the first end effector element is exerting a force against an inner surface of the second end effector element. In other words, the control system may be configured to perform step 902 whist the robotic surgical instrument is in a closed or "over-closed" configuration.

The control system may be configured to determine that the inner surface of the first end effector element is exerting a force against the inner surface of the second end effector element. Said determining may comprise: determining that the applied instrument drive forces exceed a predetermined instrument drive force threshold (e.g. -20N, where values more negative than that threshold are said to exceed that threshold); and/or determining that the applied instrument drive forces are within a predetermined range of instrument drive forces (e.g. -20N to -150N). Alternatively, or additionally, said determining may comprise: determining that the resultant spread angles predicted for the applied instrument drive forces (e.g. predicted using a kinematic equation such as Equation 4 or 5 described herein) are below a predetermined predicted spread angle threshold (e.g. -10°); and/or determining that the resultant spread angles predicted for the applied instrument drive forces are within a predetermined range of predicted spread angles (e.g. between -10° and -30°). Suitable thresholds and/or ranges may be stored by the control system 124 for each robotic surgical instrument and/or each robotic surgical instrument may store its suitable thresholds and/or ranges in a memory local to that instrument such that those thresholds and/or ranges can be provided to the control system 124 when that instrument is attached to the surgical robot arm.

In step 902, the control system may be configured to cause the instrument drive force to be varied so as to increase the force exerted by each of the first and second end effector elements on the other. Alternatively, the control system may be configured to cause the instrument drive force to be varied so as to decrease the force exerted by each of the first and second end effector elements on the other.

In step 902, the instrument drive force may be dependent on a first force applied at a first interface element (e.g. interface element 616 shown in Figure 6) for changing an angle (e.g. a yaw angle) of the first end effector element (e.g. first end effector element 208-1 shown in Figures 3 to 7) and a second force applied at a second interface element (e.g. interface element 618 shown in Figure 6) for changing an angle (e.g. a yaw angle) of the second end effector element (e.g. second end effector element 208-2 shown in Figures 3 to 7). The first force and the second force may be independently controllable. This may be the case in robotic surgical instruments in which the drive mechanism is capable of independently controlling the yaw rotation of the first and second end effector elements (e.g. as in the robotic surgical instrument 206 shown in Figures 3 to 7). In a specific example, the instrument drive force, $F$, is dependent on a sum of the first force, $f_9$, and the second force, $f_{11}$, - e.g. as defined in Equation 6:

$$F = f_9 + f_{11} \qquad (6)$$

Alternatively, in step 902, the instrument drive force may be dependent on a force applied at a single interface element for changing an angle (e.g. a yaw angle) of one or both of the first end effector element and the second end effector element. This may be the case in robotic surgical instruments in which the first and second end effector elements are not independently rotatable (e.g. yawing of both the first and second end effector elements is driven in tandem by a common pulley, gear, or rack and pinion mechanism), or in which only one of the end effector elements is rotatable whilst the other end effector element remains stationary (e.g. fixed in place relative to the articulation). In these examples, one interface element can control the spread angle between the first and second end effector elements.

In step 904, the control system is configured to predict, for each of at least two applied instrument drive forces, a resultant spread angle between the first end effector element and the second end effector element in dependence on a measured position of each of the one or more interface elements when that instrument drive force is applied. The positions of the interface elements may be measured using position sensors - such as those described with reference to Figure 5.

The control system may use a kinematic equation (e.g. Equation 4 or 5 described herein) to perform step 904. In other words, in step 904, the control system may be configured to predict, for each of the at least two applied instrument drive forces, the resultant spread angle between the first end effector element and the second end effector element in dependence on a measured position of each of the one or more interface elements when that instrument drive force is applied and the geometry of one or more components (e.g. pulleys, gears, rack and pinion mechanism etc.) of a drive transfer mechanism of the robotic surgical instrument configured for transferring force from the one or more interface elements to the first end effector element and/or the second end effector element.

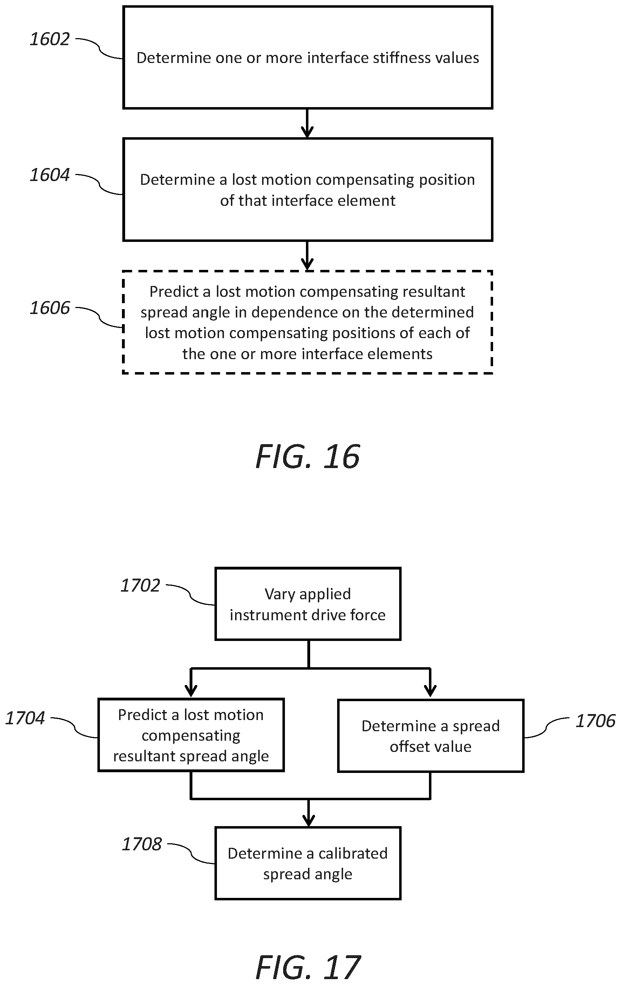

Optionally, the resultant spread angles predicted in step 904 may be partially or fully lost motion compensating - as will be described in further detail below with reference to Figure 16.

By performing steps 902 and 904, the control system can generate two or more data pairs, each data pair including a sensed applied instrument drive force and a predicted resultant spread angle. A graph can be plotted using these data pairs. Figure 8 illustrates a first experimental plot of applied instrument drive force, $F$, against predicted resultant spread angle, $\theta_s$. The experimental data is shown by solid line 800. It is noted that Figure 8 shows "negative" resultant spread angles being predicted at certain instrument drive forces. Whilst, in reality, it is not possible to achieve negative spread angles using robotic surgical instruments of the type described herein - e.g. because the first and second end effector elements come into contact at spread angles of 0° and cannot rotate further past/through one another - negative spread angles can be predicted by the kinematic equations when the robotic surgical instrument is in a closed configuration and further "closing" instrument drive forces are being applied at the one or more instrument interface elements such that their measured position(s) change (e.g. owing to lost motion, such as by cable stretch in the drive transfer mechanism).

In Figure 8, the experimental plot can be roughly divided into two portions, each being approximately linear - a first portion on the left-hand-side having a higher gradient, and a second portion on the right-hand-side having a lower gradient. The first portion corresponds to data pairs generated whist an inner surface of the first end effector element is exerting a force against an inner surface of the second end effector element - e.g. whilst the robotic surgical instrument is in a closed or "over-closed" configuration. The second portion corresponds to data pairs generated whist the robotic surgical instrument is in an open configuration.

The data pairs generated by performing steps 902 and 904 as described herein would appear in the first portion of an experimental plot such as the one shown in Figure 8, as step 902 is performed whist an inner surface of the first end effector element is exerting a force against an inner surface of the second end effector element.

In step 906, the control system is configured to fit a linear relationship to the at least two applied instrument drive force and predicted resultant spread angle data pairs. An example linear relationship is shown by dashed line 802 in Figure 8. At least two force-spread data pairs are used to fit a linear relationship. Greater accuracy can be achieved by fitting the linear relationship to a greater number of data pairs, although doing so can also increase the computational load for the control system. In an example, the linear relationship can be fit using a first data pair having a first applied instrument drive force value above an upper force threshold and a second data pair having a second applied instrument drive force value below a lower force threshold. Greater accuracy can be achieved by setting the upper and lower force thresholds sufficiently far apart (e.g. at least 20N apart) such that the first and second data pairs do not have similar applied instrument drive force values.

The linear relationship may be defined by Equation 7, where k is a gradient of the linear relationship, and C is a y-intercept value.

$$F = k\theta_s + C \qquad (7)$$

In step 908, the control system is configured to determine a spread offset value. Determining the spread offset value comprises approximating, using the linear relationship fitted in step 906, a predicted spread angle when the applied instrument drive force would equal a predetermined force value. Said predicted spread angle may be determined as the spread offset value.

The predetermined force value may be the force value expected when the robotic surgical instrument is in a "zero configuration". When the control system is configured to cause the instrument drive force to be varied to close the robotic surgical instrument from an open configuration, the zero configuration is the configuration in which an inner surface of the first end effector element first contacts an inner surface of the second end effector element. When the control system is configured to cause the instrument drive force to be varied to open the robotic surgical instrument from a closed configuration, the zero configuration is the configuration in which an inner surface of the first end effector element last contacts an inner surface of the second end effector element. Put another way, the zero configuration may be defined as a configuration in which an inner surface of the first end effector element is touching, but not exerting any force on, an inner surface of the second end effector element. As such, the predetermined force value may be zero. In other examples, the predetermined force may be greater than or less than zero - e.g. so as to account for the instrument drive force required to overcome frictional or gravitational forces experienced by the drive mechanism or the drive transfer mechanism.

Conceptually, approximating the predicted spread angle when the applied instrument drive force would equal the predetermined force value can comprise extrapolating the linear relationship to the predetermined force value. This is illustrated in Figure 8, where linear relationship 802 is extrapolated to a predetermined force value of zero (i.e. $F = 0$ on the y-axis). In this example, a predicted spread angle of -4.4° is approximated when the applied instrument drive force would equal zero. The predicted spread angle of -4.4° can be determined to be the spread offset value. This means that, in this Figure 8 example, the control system has determined that the robotic surgical instrument was in a "zero configuration" when the kinematic equations were predicting a spread angle of -4.4° (i.e. an over-closed configuration). It follows that, when the kinematic equations were predicting a spread angle of 0°, the robotic surgical instrument was in fact in an open configuration (e.g. with a spread angle of approximately +4.4°).

In step 910, the control system is configured to control the surgical robot arm to drive the robotic surgical instrument in dependence on the spread offset value. The control system may be configured to use the spread offset value to adjust subsequently predicted resultant spread angles. For example, the control system may be configured to subtract the spread offset value from the output of the kinematic equations used to predict a resultant spread angle for an applied instrument drive force so as to determine a calibrated spread angle. In this way, the control system can control the surgical robot arm such that an appropriate instrument drive force is applied in order that the robotic surgical instrument is driven to a configuration (e.g. a spread angle) that more closely matches the configuration (e.g. the spread angle) requested by the input received at the surgeon input device. In a specific example, the control system may be configured to use the spread offset value to determine a calibrated zero configuration for the robotic surgical instrument. In this way, the control system can control the surgical robot arm such that an appropriate instrument drive force is applied in order that the robotic surgical instrument is driven to the calibrated zero configuration when the input received at the surgeon input device requests that the robotic surgical instrument is in the zero configuration. For example, referring to Figure 8, in response to receiving an input at the surgeon input device requesting that the robotic surgical instrument is in the zero configuration (e.g. having a spread angle of 0°), the control system may control the surgical robot arm to apply an appropriate instrument drive force to drive the robotic surgical instrument to a predicted spread angle of -4.4° (e.g. as subtracting the spread offset value, -4.4°, from the predicted spread angle, -4.4°, gives an adjusted spread angle of 0°). That is, in this example, the control system can cause the one or more interface elements to be driven to respective measured positions that, when input to the kinematic equations, output a predicted spread angle of -4.4°.

Configuring the control system (e.g. control system 124 shown in Figure 1) to perform steps 902, 904, 906, 908 and 910 can improve the surgeon's perception that the movements of the robotic surgical instrument are accurately reflecting the inputs they provide to the surgeon input device.

The control system can be configured to perform steps 902, 904, 906 and 908 as part of a calibration process prior to using a robotic surgical instrument to perform aspects of a minimally invasive surgical procedure. For example, such a calibration process may be performed prior to beginning a minimally invasive surgical procedure, and/or each time a new robotic surgical instrument is attached to the surgical robot arm during the minimally invasive surgical procedure.

Alternatively, or additionally, the control system can be configured to perform steps 902, 904, 906 and 908 iteratively during a minimally invasive surgical procedure, so as to iteratively update the determined spread offset value. For example, the control system may be configured to automatically perform steps 902, 904, 906 and 908 each time it is determined that an inner surface of the first end effector element is exerting a force against an inner surface of the second end effector element during a minimally invasive surgical procedure. This approach of iteratively updating the determined spread offset value can advantageously take account of "wear and tear" of the robotic surgical instrument during the minimally invasive surgical procedure. In these examples, in order to mitigate against the control system determining a spread offset value whilst the robotic surgical instrument is grasping an object between its end effector elements (e.g. whilst robotic surgical instrument is in an open configuration) the control system may be configured to determine the spread offset value in dependence on a range of permissible spread offset values. For example, the range of permissible spread offset values may be - 15° to +15°. The spread offset value may not be updated if the control system determines a spread offset value outside of that permissible range.

### Concept 1 - interface position offset value

In the set of steps described with reference to Figure 9, step 904 involves predicting a resultant spread angle in dependence on a measured position of each of the one or more interface elements. The following steps of Figure 9 are performed in dependence on that predicted resultant spread angle so as to control the surgical robot arm to drive the robotic surgical instrument in dependence on a spread offset value. In examples where a single interface element can control the spread angle between the first and second end effector elements, this "predicting" step need not be performed. This is because the measured position of that interface element will be indicative of a predicted spread angle between the first end effector element and the second end effector element. By contrast, in examples where the spread angle is dependent on the position of two or more interface elements, the position of any one of those interface elements alone is not necessarily indicative of the spread angle.

Figure 10 illustrates a first set of steps that can be performed by a control system (e.g. control system 124 shown in Figure 1) for controlling a surgical robot arm (e.g. surgical robot arm 101 shown in Figure 1) in dependence on a determined interface position offset value. The surgical robot arm has a drive mechanism configured for driving a robotic surgical instrument. The robotic surgical instrument comprises a first end effector element and a second end effector element. The drive mechanism comprises an interface element (e.g. a single interface element, or only one interface element) configured to interface with the robotic surgical instrument for controlling a spread angle between the first end effector element and the second end effector element.

Preferably, the control system can be configured to perform the set of steps described with reference to Figure 10 when a robotic surgical instrument in which the first and second end effector elements are not independently rotatable (e.g. yawing of both the first and second end effector elements is driven in tandem by a common pulley, gear, or rack and pinion mechanism), or in which only one of the end effector elements is rotatable whilst the other end effector element remains stationary (e.g. fixed in place relative to the articulation), is attached to the surgical robot arm.

In step 1002, the control system is configured to cause an instrument drive force applied at the interface element to be varied. In step 1002, the instrument drive force is applied at a single interface element for changing an angle (e.g. a yaw angle) of one or both of the first end effector element and the second end effector element. The applied forces may be measured using a force sensor - such as the force sensor described with reference to Figure 5. The control system is configured to perform step 1002 whilst an inner surface of the first end effector element is exerting a force against an inner surface of the second end effector element. In other words, the control system may be configured to perform step 1002 whist the robotic surgical instrument is in a closed or "over-closed" configuration.

In step 1002, the control system may be configured to determine that the inner surface of the first end effector element is exerting a force against the inner surface of the second end effector element. Said determining may comprise: determining that the applied instrument drive forces exceed a predetermined instrument drive force threshold (e.g. -20N, where values more negative than that threshold are said to exceed that threshold); and/or determining that the applied instrument drive forces are within a predetermined range of instrument drive forces (e.g. -20N to -150N). Alternatively, or additionally, said determining may comprise: determining that resultant spread angles predicted for the applied instrument drive forces (e.g. predicted using a suitable kinematic equation) are below a predetermined predicted spread angle threshold (e.g. -10°); and/or determining that resultant spread angles predicted for the applied instrument drive forces are within a predetermined range of predicted spread angles (e.g. between -10° and -30°). Suitable thresholds and/or ranges may be stored by the control system 124 for each robotic surgical instrument and/or each robotic surgical instrument may store its suitable thresholds and/or ranges in a memory local to that instrument such that those thresholds and/or ranges can be provided to the control system 124 when that instrument is attached to the surgical robot arm.

In step 1002, the control system may be configured to cause the instrument drive force to be varied so as to increase the force exerted by each of the first and second end effector elements on the other. Alternatively, the control system may be configured to cause the instrument drive force to be varied so as to decrease the force exerted by each of the first and second end effector elements on the other.

In step 1004, the control system is configured to, for each of at least two applied instrument drive forces, measure a position of the interface element when that instrument drive force is applied which is indicative of a predicted spread angle between the first end effector element and the second end effector element. The position of the interface element may be measured using a position sensor - such as the position sensor described with reference to Figure 5.

Optionally, the positions measured in step 1004 may be partially or fully lost motion compensating - as will be described in further detail below with reference to Figure 16.

By performing steps 1002 and 1004, the control system can generate two or more data pairs, each data pair including a sensed applied instrument drive force and a measured position of the interface element. A graph can be plotted using these data pairs.

In step 1006, the control system is configured to fit a linear relationship to the at least two applied instrument drive force and measured position data pairs. At least two force-position data pairs are used to fit a linear relationship. Greater accuracy can be achieved by fitting the linear relationship to a greater number of data pairs, although doing so can also increase the computational load for the control system. In an example, the linear relationship can be fit using a first data pair having a first applied instrument drive force value above an upper force threshold and a second data pair having a second applied instrument drive force value below a lower force threshold. Greater accuracy can be achieved by setting the upper and lower force thresholds sufficiently far apart (e.g. at least 20N apart) such that the first and second data pairs do not have similar applied instrument drive force values.

The linear relationship may be defined by Equation 8, where $q$ is the measured position of the interface element, $k$ is a gradient of the linear relationship, and C is a y-intercept value.

$$F = kq + C \qquad (8)$$

In step 1008, the control system is configured to determine an interface position offset value. Determining the interface position offset value comprises approximating, using the linear relationship fitted in step 1006, an interface position when the applied instrument drive force would equal a predetermined force value. Said interface position angle may be determined as the interface position offset value. Conceptually, approximating the interface position when the applied instrument drive force would equal the predetermined force value can comprise extrapolating the linear relationship to the predetermined force value.

The predetermined force value may be the force value expected when the robotic surgical instrument is in a "zero configuration", as defined herein. The predetermined force value may be zero. In other examples, the predetermined force may be greater than or less than zero - e.g. so as to account for the instrument drive force required to overcome frictional or gravitational forces experienced by the drive mechanism or the drive transfer mechanism.

In step 1010, the control system is configured to control the surgical robot arm to drive the robotic surgical instrument in dependence on the interface position offset value. The control system may be configured to use the interface position offset value to adjust subsequently measured interface positions that are to be input to the kinematic equations used to predict a resultant spread angle for an applied instrument drive force. For example, the control system may be configured to subtract the interface position offset value from the measured interface positions so as to determine calibrated interface positions to be input to the kinematic equations. In this way, the control system can control the surgical robot arm such that an appropriate instrument drive force is applied in order that the robotic surgical instrument is driven to a configuration (e.g. a spread angle) that more closely matches the configuration (e.g. the spread angle) requested by the input received at the surgeon input device. In a specific example, the control system may be configured to use the interface position offset value to determine a calibrated zero configuration for the robotic surgical instrument. In this way, the control system can control the surgical robot arm such that an appropriate instrument drive force is applied in order that the robotic surgical instrument is driven to the calibrated zero configuration when the input received at the surgeon input device requests that the robotic surgical instrument is in the zero configuration. For example, the interface position offset value may be used to store a calibrated zero position for the interface element in the control system.

Configuring the control system (e.g. control system 124 shown in Figure 1) to perform steps 1002, 1004, 1006, 1008 and 1010 can improve the surgeon's perception that the movements of the robotic surgical instrument are accurately reflecting the inputs they provide to the surgeon input device.

The control system can be configured to perform steps 1002, 1004, 1006 and 1008 as part of a calibration process prior to using a robotic surgical instrument to perform aspects of a minimally invasive surgical procedure. For example, such a calibration process may be performed prior to beginning a minimally invasive surgical procedure, and/or each time a new robotic surgical instrument is attached to the surgical robot arm during the minimally invasive surgical procedure.

Alternatively, or additionally, the control system can be configured to perform steps 1002, 1004, 1006 and 1008 iteratively during a minimally invasive surgical procedure, so as to iteratively update the determined interface position offset value. For example, the control system may be configured to automatically perform steps 1002, 1004, 1006 and 1008 each time it is determined that an inner surface of the first end effector element is exerting a force against an inner surface of the second end effector element during a minimally invasive surgical procedure. This approach of iteratively updating the determined interface position offset value can advantageously take account of "wear and tear" of the robotic surgical instrument during the minimally invasive surgical procedure. In these examples, in order to mitigate against the control system determining an interface position offset value whilst the robotic surgical instrument is grasping an object between its end effector elements (e.g. whilst robotic surgical instrument is in an open configuration) the control system may be configured to determine the interface position offset value in dependence on a range of permissible interface position offset values. For example, the range of permissible interface position offset values may be indicative of predicted spread angles of -15° to +15° - e.g., for an example robotic surgical instrument, the range of permissible interface position offset values may be -1.2mm to +1.2mm as defined relative to the neutral (e.g. resting) position of the interface element, and/or -10% to +10% of the total possible travel of an interface element relative to its neutral (e.g. resting) position (e.g. at 50%, but not necessarily). The interface position offset value may not be updated if the control system determines an interface position offset value outside of that permissible range.

*Concept 2 - spread offset value*

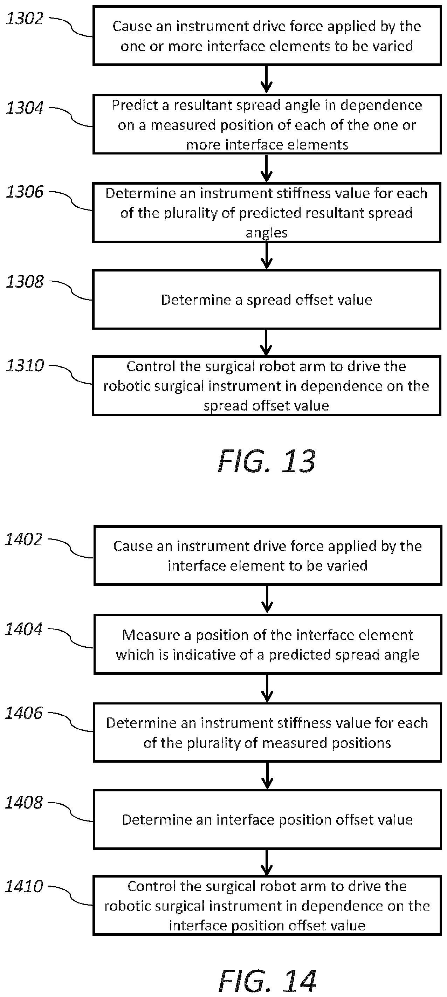

Figure 13 illustrates a second set of steps that can be performed by a control system (e.g. control system 124 shown in Figure 1) for controlling a surgical robot arm (e.g. surgical robot arm 101 shown in Figure 1) in dependence on a determined spread offset value. The surgical robot arm has a drive mechanism configured for driving a robotic surgical instrument. The robotic surgical instrument comprises a first end effector element and a second end effector element. The drive mechanism comprises one or more interface elements configured to interface with the robotic surgical instrument for controlling a spread angle between the first end effector element and the second end effector element.

In step 1302, the control system is configured to cause an instrument drive force applied at the one or more interface elements to be varied. The applied forces may be measured using force sensors - such as those described with reference to Figure 5.

In step 1302, the control system may be configured to cause the instrument drive force to be varied so as to close the robotic surgical instrument from an open configuration. Alternatively, the control system may be configured to cause the instrument drive force to be varied so as to open the robotic surgical instrument from a closed configuration.

In step 1302, the instrument drive force may be dependent on a first force applied at a first interface element (e.g. interface element 616 shown in Figure 6) for changing an angle (e.g. a yaw angle) of the first end effector element (e.g. first end effector element 208-1 shown in Figures 3 to 7) and a second force applied at a second interface element (e.g. interface element 618 shown in Figure 6) for changing an angle (e.g. a yaw angle) of the second end effector element (e.g. second end effector element 208-2 shown in Figures 3 to 7). The first force and the second force may be independently controllable. This may be the case in robotic surgical instruments in which the drive mechanism is capable of independently controlling the yaw rotation of the first and second end effector elements (e.g. as in the robotic surgical instrument 206 shown in Figures 3 to 7). In a specific example, the instrument drive force, $F$, is dependent on a sum of the first force, $f_9$, and the second force, $f_{11}$, - e.g. as defined in Equation 6 above.

Alternatively, in step 1302, the instrument drive force may be dependent on a force applied at a single interface element for changing an angle (e.g. a yaw angle) of one or both of the first end effector element and the second end effector element. This may be the case in robotic surgical instruments in which the first and second end effector elements are not independently rotatable (e.g. yawing of both the first and second end effector elements is driven in tandem by a common pulley, gear, or rack and pinion mechanism), or in which only one of the end effector elements is rotatable whilst the other end effector element remains stationary (e.g. fixed in place relative to the articulation). In these examples, one interface element can control the spread angle between the first and second end effector elements.

In step 1304, the control system is configured to predict, for each of a plurality of applied instrument drive forces, a resultant spread angle between the first end effector element and the second end effector element in dependence on a measured position of each of the one or more interface elements when that instrument drive force is applied. The positions of the interface elements may be measured using position sensors - such as those described with reference to Figure 5.

The control system may use a kinematic equation (e.g. Equation 4 or 5 described herein) to perform step 1304. In other words, in step 1304, the control system may be configured to predict, for each of the plurality of applied instrument drive forces, the resultant spread angle between the first end effector element and the second end effector element in dependence on a measured position of each of the one or more interface elements when that instrument drive force is applied and the geometry of one or more components (e.g. pulleys, gears, rack and pinion mechanism etc.) of a drive transfer mechanism of the robotic surgical instrument configured for transferring force from the one or more interface elements to the first end effector element and/or the second end effector element.

Optionally, the resultant spread angles predicted in step 1304 may be partially or fully lost motion compensating - as will be described in further detail below with reference to Figure 16.

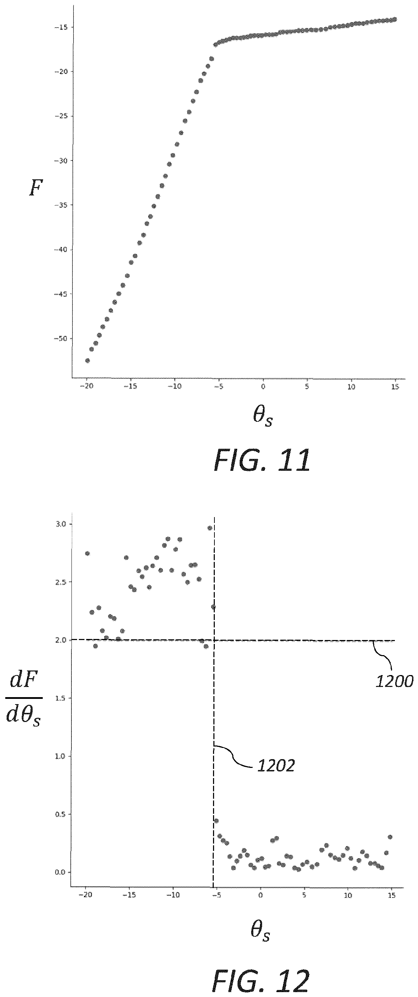

By performing steps 1302 and 1304, the control system can generate a plurality of data pairs, each data pair including a sensed applied instrument drive force and a predicted resultant spread angle. A graph can be plotted using these data pairs. Figure 11 illustrates a second experimental plot of applied instrument drive force, $F$, against predicted resultant spread angle, $\theta_s$. In Figure 11, the experimental plot can be roughly divided into two portions, each being approximately linear - a first portion on the left-hand-side having a higher gradient, and a second portion on the right-hand-side having a lower gradient. The first portion corresponds to data pairs generated whist an inner surface of the first end effector element is exerting a force against an inner surface of the second end effector element - e.g. whilst the robotic surgical instrument is in a closed or "over-closed" configuration. The second portion corresponds to data pairs generated whist the robotic surgical instrument is in an open configuration. As such, it is desirable to determine where the boundary between the first and second portions lies, as this boundary represents the transition between the closed and open configurations of the robotic surgical instrument.

In step 1306, the control system is configured to determine, for each of the plurality of predicted resultant spread angles, an instrument stiffness value using a derivative of the applied instrument drive force with respect to the predicted resultant spread angle at that predicted resultant spread angle. For example, the derivative may be the first derivative of the applied instrument drive force with respect to the predicted resultant spread angle - as represented by Equation 9:

$$\frac{dF}{d\theta_s} \qquad (9)$$

In order to mitigate against the control system performing step 1306 whilst the robotic surgical instrument is grasping an object between its end effector elements (e.g. whilst robotic surgical instrument the in an open configuration), the control system may be configured to determine an instrument stiffness value for each of the plurality of predicted resultant spread angles in response to determining that: the plurality of predicted resultant spread angles are within a predetermined range of spread angles (e.g. between 20° and -20°); the plurality of predicted resultant spread angles are predicted for applied instrument drive forces within a predetermined range of instrument drive forces (e.g. between -10N and -50N); and/or the predicted resultant spread angles of the plurality of predicted resultant spread angles are changing at a rate faster than a predetermined threshold (e.g. -30°/s, which represents robotic surgical instrument closing at a rate of 30°/s). Suitable thresholds and/or ranges may be stored by the control system 124 for each robotic surgical instrument and/or each robotic surgical instrument may store its suitable thresholds and/or ranges in a memory local to that instrument such that those thresholds and/or ranges can be provided to the control system 124 when that instrument is attached to the surgical robot arm.

Figure 12 illustrates plot of the first derivative of applied instrument drive force with respect to predicted resultant spread angle, $\frac{dF}{d\theta_s}$, against resultant spread angle, $\theta_s$. The data shown in Figure 12 has been derived by determining instrument stiffness values for the data shown in Figure 11.

In step 1308, the control system is configured to determine a spread offset value in dependence on the plurality of determined instrument stiffness values. Determining the spread offset value may comprise approximating a predicted spread angle at which a step change occurs in the determined instrument stiffness values. For example, such a step change can be seen in Figure 12. Said predicted spread angle may be determined as the spread offset value.

In an example, the control system may be configured to cause the instrument drive force to be varied to close the robotic surgical instrument from an open configuration. Referring to Figures 11 and 12, closing the robotic surgical instrument from an open configuration would cause data to be generated, chronologically, from the right-hand-side of those plots to left-hand-side (e.g. as the robotic surgical instrument closes from positive predicted spread angles to negative spread angles). In this example, determining the spread offset value can comprise approximating a predicted spread angle at which the determined instrument stiffness value first reaches or exceeds a predetermined threshold. Said predicted spread angle may be determined as the spread offset value. In Figure 12, this predetermined threshold is at an instrument stiffness value, $\frac{dF}{d\theta_S}$, of 2 N/°, as illustrated by dashed line 1200. A suitable threshold may be stored by the control system 124 for each robotic surgical instrument and/or each robotic surgical instrument may store its suitable threshold in a memory local to that instrument such that that threshold can be provided to the control system 124 when that instrument is attached to the surgical robot arm. The approximate predicted spread angle, $\theta_S$, at which the determined instrument stiffness value first reaches or exceeds this predetermined threshold is -5.5°, as illustrated by dashed line 1202. The predicted spread angle of -5.5° can be determined to be the spread offset value. This means that, in this Figure 12 example, the control system has determined that the robotic surgical instrument was in a "zero configuration", as defined herein, when the kinematic equations were predicting a spread angle of -5.5° (i.e. an over-closed configuration). It follows that, when the kinematic equations were predicting a spread angle of 0°, the robotic surgical instrument was in fact in an open configuration (e.g. with a spread angle of approximately +5.5°).

In an alternative example, the control system may be configured to cause the instrument drive force to be varied to open the robotic surgical instrument from a closed configuration. Referring to Figures 11 and 12, opening the robotic surgical instrument from a closed configuration would cause data to be generated, chronologically, from the left-hand-side of those plots to right-hand-side (e.g. as the robotic surgical instrument opens from negative predicted spread angles to positive spread angles). In this example, determining the spread offset value can comprise approximating a predicted spread angle at which the determined instrument stiffness value first reaches or falls below a predetermined threshold (e.g. 0.5 N/°). A suitable threshold may be stored by the control system 124 for each robotic surgical instrument and/or each robotic surgical instrument may store its suitable threshold in a memory local to that instrument such that that threshold can be provided to the control system 124 when that instrument is attached to the surgical robot arm. Said predicted spread angle may be determined as the spread offset value.

In step 1310, the control system is configured to control the surgical robot arm to drive the robotic surgical instrument in dependence on the spread offset value. The control system may be configured to use the spread offset value to adjust subsequently predicted resultant spread angles. For example, the control system may be configured to subtract the spread offset value from the output of the kinematic equations used to predict a resultant spread angle for an applied instrument drive force so as to determine a calibrated spread angle. In this way, the control system can control the surgical robot arm such that an appropriate instrument drive force is applied in order that the robotic surgical instrument is driven to a configuration (e.g. a spread angle) that more closely matches the configuration (e.g. the spread angle) requested by the input received at the surgeon input device. In a specific example, the control system may be configured to use the spread offset value to determine a calibrated zero configuration for the robotic surgical instrument. In this way, the control system can control the surgical robot arm such that an appropriate instrument drive force is applied in order that the robotic surgical instrument is driven to the calibrated zero configuration when the input received at the surgeon input device requests that the robotic surgical instrument is in the zero configuration. For example, referring to Figure 12, in response to receiving an input at the surgeon input device requesting that the robotic surgical instrument is in the zero configuration (e.g. having a spread angle of 0°), the control system may control the surgical robot arm to apply an appropriate instrument drive force to drive the robotic surgical instrument to a predicted spread angle of -5.5° (e.g. as subtracting the spread offset value, - 5.5°, from the predicted spread angle, -5.5°, gives an adjusted spread angle of 0°). That is, in this example, the control system can cause the one or more interface elements to be driven to respective measured positions that, when input to the kinematic equations, output a predicted spread angle of - 5.5°.

Configuring the control system (e.g. control system 124 shown in Figure 1) to perform steps 1302, 1304, 1306, 1308 and 1310 can improve the surgeon's perception that the movements of the robotic surgical instrument are accurately reflecting the inputs they provide to the surgeon input device.

The control system can be configured to perform steps 1302, 1304, 1306 and 1308 as part of a calibration process prior to using a robotic surgical instrument to perform aspects of a minimally invasive surgical procedure. For example, such a calibration process may be performed prior to beginning a minimally invasive surgical procedure, and/or each time a new robotic surgical instrument is attached to the surgical robot arm during the minimally invasive surgical procedure.

Alternatively, or additionally, the control system can be configured to perform steps 1302, 1304, 1306 and 1308 iteratively during a minimally invasive surgical procedure, so as to iteratively update the determined spread offset value. For example, the control system may be configured to automatically perform steps 1306 and 1308 each time the conditions for determining an instrument stiffness value, as described herein with reference to step 1306, are met during a minimally invasive surgical procedure. This approach of iteratively updating the determined spread offset value can advantageously take account of "wear and tear" of the robotic surgical instrument during the minimally invasive surgical procedure. In these examples, in order to mitigate against the control system determining a spread offset value whilst the robotic surgical instrument is grasping an object between its end effector elements (e.g. whilst robotic surgical instrument the in an open configuration) the control system may be configured to determine the spread offset value in dependence on a range of permissible spread offset values. For example, the range of permissible spread offset values may be -15° to +15°. The spread offset value may not be updated if the control system determines a spread offset value outside of that permissible range.

*Concept 2 - interface position offset value*

In the set of steps described with reference to Figure 13, step 1304 involves predicting a resultant spread angle in dependence on a measured position of each of the one or more interface elements. The following steps of Figure 13 are performed in dependence on that predicted resultant spread angle so as to control the surgical robot arm to drive the robotic surgical instrument in dependence on a spread offset value. In examples where a single interface element can control the spread angle between the first and second end effector elements, this "predicting" step need not be performed. This is because the measured position of that interface element will be indicative of a predicted spread angle between the first end effector element and the second end effector element. By contrast, in examples where the spread angle is dependent on the position of two or more interface elements, the position of any one of those interface elements alone is not necessarily indicative of the spread angle.

Figure 14 illustrates a second set of steps that can be performed by a control system (e.g. control system 124 shown in Figure 1) for controlling a surgical robot arm (e.g. surgical robot arm 101 shown in Figure 1) in dependence on a determined interface position offset value. The surgical robot arm has a drive mechanism configured for driving a robotic surgical instrument. The robotic surgical instrument comprises a first end effector element and a second end effector element. The drive mechanism comprises an interface element (e.g. a single interface element, or only one interface element) configured to interface with the robotic surgical instrument for controlling a spread angle between the first end effector element and the second end effector element.

Preferably, the control system can be configured to perform the set of steps described with reference to Figure 14 when a robotic surgical instrument in which the first and second end effector elements are not independently rotatable (e.g. yawing of both the first and second end effector elements is driven in tandem by a common pulley, gear, or rack and pinion mechanism), or in which only one of the end effector elements is rotatable whilst the other end effector element remains stationary (e.g. fixed in place relative to the articulation), is attached to the surgical robot arm.

In step 1402, the control system is configured to cause an instrument drive force applied at the interface element to be varied. In step 1402, the instrument drive force is applied at a single interface element for changing an angle (e.g. a yaw angle) of one or both of the first end effector element and the second end effector element. The applied forces may be measured using a force sensor - such as the force sensor described with reference to Figure 5.

In step 1402, the control system may be configured to cause the instrument drive force to be varied so as to close the robotic surgical instrument from an open configuration. Alternatively, the control system may be configured to cause the instrument drive force to be varied so as to open the robotic surgical instrument from a closed configuration.

In step 1404, the control system is configured to, for each of a plurality of applied instrument drive forces, measure a position of the interface element when that instrument drive force is applied which is indicative of a predicted spread angle between the first end effector element and the second end effector element. The position of the interface element may be measured using a position sensor - such as the position sensor described with reference to Figure 5.

Optionally, the positions measured in step 1404 may be partially or fully lost motion compensating - as will be described in further detail below with reference to Figure 16.

In step 1406, the control system is configured to determine, for each of the plurality of measured positions, an instrument stiffness value using a derivative of the applied instrument drive force with respect to the measured position at that measured position. For example, the derivative may be the first derivative of the applied instrument drive force with respect to the measured position - as represented by Equation 10, where $q$ is the measured position of the interface element:

$$\frac{dF}{dq} \qquad (10)$$

In order to mitigate against the control system performing step 1406 whilst the robotic surgical instrument is grasping an object between its end effector elements (e.g. whilst robotic surgical instrument the in an open configuration), the control system may be configured to determine an instrument stiffness value for each of the plurality of measured positions in response to determining that: the plurality of measured positions are within a predetermined range of measured positions (e.g. a predetermined range of measured positions that are indicative of predicted spread angles of between -20° and +20° - e.g., for an example robotic surgical instrument, the predetermined range of measured positions may be -2mm to +2mm); the plurality of measured positions are measured for applied instrument drive forces within a predetermined range of instrument drive forces (e.g. between -10N and -50N); and/or the measured positions of the plurality of measured positions are changing at a rate faster than a predetermined threshold (e.g. a predetermined threshold rate indicative of the robotic surgical instrument closing at a rate of 30°/s). Suitable thresholds and/or ranges may be stored by the control system 124 for each robotic surgical instrument and/or each robotic surgical instrument may store its suitable thresholds and/or ranges in a memory local to that instrument such that those thresholds and/or ranges can be provided to the control system 124 when that instrument is attached to the surgical robot arm.

In step 1408, the control system is configured to determine an interface position offset value in dependence on the plurality of determined instrument stiffness values. Determining the interface position offset value may comprise approximating a measured interface position at which a step change occurs in the determined instrument stiffness values. Said measured interface position may be determined as the interface position offset value.

In an example, the control system may be configured to cause the instrument drive force to be varied to close the robotic surgical instrument from an open configuration. In this example, determining the interface position offset value comprises approximating a measured position at which the determined instrument stiffness value first reaches or exceeds a predetermined threshold (e.g. 20 N/mm). A suitable threshold may be stored by the control system 124 for each robotic surgical instrument and/or each robotic surgical instrument may store its suitable threshold in a memory local to that instrument such that that threshold can be provided to the control system 124 when that instrument is attached to the surgical robot arm. Said measured interface position may be determined as the interface position offset value.

In an alternative example, the control system may be configured to cause the instrument drive force to be varied to open the robotic surgical instrument from a closed configuration. In this example, determining the interface position offset value comprises approximating a measured position at which the determined instrument stiffness value first reaches or falls below a predetermined threshold (e.g. 5 N/mm). A suitable threshold may be stored by the control system 124 for each robotic surgical instrument and/or each robotic surgical instrument may store its suitable threshold in a memory local to that instrument such that that threshold can be provided to the control system 124 when that instrument is attached to the surgical robot arm. Said measured interface position may be determined as the interface position offset value.

In step 1410, the control system is configured to control the surgical robot arm to drive the robotic surgical instrument in dependence on the interface position offset value. The control system may be configured to use the interface position offset value to adjust subsequently measured interface positions that are to be input to the kinematic equations used to predict a resultant spread angle for an applied instrument drive force. For example, the control system may be configured to subtract the interface position offset value from the measured interface positions so as to determine calibrated interface positions to be input to the kinematic equations. In this way, the control system can control the surgical robot arm such that an appropriate instrument drive force is applied in order that the robotic surgical instrument is driven to a configuration (e.g. a spread angle) that more closely matches the configuration (e.g. the spread angle) requested by the input received at the surgeon input device. In a specific example, the control system may be configured to use the interface position offset value to determine a calibrated zero configuration for the robotic surgical instrument. In this way, the control system can control the surgical robot arm such that an appropriate instrument drive force is applied in order that the robotic surgical instrument is driven to the calibrated zero configuration when the input received at the surgeon input device requests that the robotic surgical instrument is in the zero configuration.

Configuring the control system (e.g. control system 124 shown in Figure 1) to perform steps 1402, 1404, 1406, 1408 and 1410 can improve the surgeon's perception that the movements of the robotic surgical instrument are accurately reflecting the inputs they provide to the surgeon input device.

The control system can be configured to perform steps 1402, 1404, 1406 and 1408 as part of a calibration process prior to using a robotic surgical instrument to perform aspects of a minimally invasive surgical procedure. For example, such a calibration process may be performed prior to beginning a minimally invasive surgical procedure, and/or each time a new robotic surgical instrument is attached to the surgical robot arm during the minimally invasive surgical procedure.

Alternatively, or additionally, the control system can be configured to perform steps 1402, 1404, 1406 and 1408 iteratively during a minimally invasive surgical procedure, so as to iteratively update the determined interface position offset value. For example, the control system may be configured to automatically perform steps 1406 and 1408 each time the conditions for determining an instrument stiffness value, as described herein with reference to step 1406, are met during a minimally invasive surgical procedure. This approach of iteratively updating the determined interface position offset value can advantageously take account of "wear and tear" of the robotic surgical instrument during the minimally invasive surgical procedure. In these examples, in order to mitigate against the control system determining an interface position offset value whilst the robotic surgical instrument is grasping an object between its end effector elements (e.g. whilst robotic surgical instrument the in an open configuration) the control system may be configured to determine the interface position offset value in dependence on a range of permissible interface position offset values. For example, the range of permissible interface position offset values may be indicative of predicted spread angles of -15° to +15° - e.g., for an example robotic surgical instrument, the range of permissible interface position offset values may be -1.2mm to +1.2mm as defined relative to the neutral (e.g. resting) position of the interface element, and/or -10% to +10% of the total possible travel of an interface element relative to its neutral (e.g. resting) position (e.g. at 50%, but not necessarily). The interface position offset value may not be updated if the control system determines an interface position offset value outside of that permissible range.

*Concept 3 - spread offset value or interface position offset value*

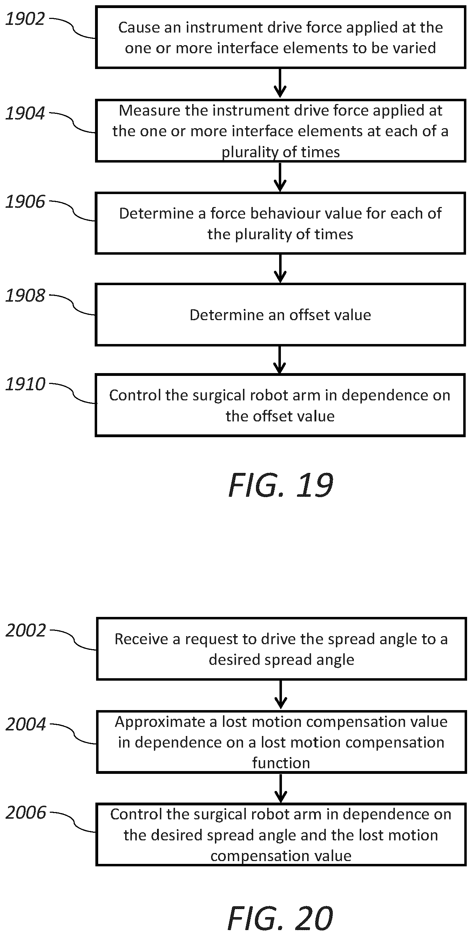

Figure 19 illustrates another set of steps that can be performed by a control system (e.g. control system 124 shown in Figure 1) for controlling a surgical robot arm (e.g. surgical robot arm 101 shown in Figure 1) in dependence on a determined offset value. The determined offset value may be a spread offset value or an interface position offset value. The surgical robot arm has a drive mechanism configured for driving a robotic surgical instrument. The robotic surgical instrument comprises a first end effector element and a second end effector element. The drive mechanism comprises one or more interface elements configured to interface with the robotic surgical instrument for controlling a spread angle between the first end effector element and the second end effector element.

In step 1902, the control system is configured to cause an instrument drive force applied at the one or more interface elements to be varied. In step 1902, the control system may be configured to cause the instrument drive force to be varied so as to close the robotic surgical instrument from an open configuration. Alternatively, or additionally, the control system may be configured to cause the instrument drive force to be varied so as to open the robotic surgical instrument from a closed configuration. For example, the control system may be configured to cause the instrument drive force to be varied such that the robotic surgical instrument performs a "calibration routine". That calibration routine may comprise closing the robotic surgical instrument from an open configuration at least once and opening the robotic surgical instrument from a closed configuration at least once. In a specific example, the calibration routine may comprise: opening the robotic surgical instrument from a zero configuration (e.g. a commanded spread angle of 0°) to an open configuration (e.g. a commanded spread angle of 30°); subsequently closing the robotic surgical instrument from that open configuration to a closed configuration (e.g. a commanded spread angle of -60°); subsequently opening the robotic surgical instrument from that closed configuration to an open configuration (e.g. a commanded spread angle of 30°); and subsequently closing the robotic surgical instrument from that open configuration to a closed configuration (e.g. a commanded spread angle of -60°).

In step 1902, the control system may be configured to cause the instrument drive force applied at the one or more interface elements to be varied such that each of the one or more interface elements move at a substantially constant velocity (e.g. a constant velocity). For example, the constant velocity may be $\pm 6$mm/s.

In step 1904, the control system is configured to measure the instrument drive force applied at the one or more interface elements at each of a plurality of times. The applied forces may be measured using force sensors - such as those described with reference to Figure 5.

In a first example, the measured instrument drive force may be dependent on a first force applied at a first interface element (e.g. interface element 616 shown in Figure 6) for changing an angle (e.g. a yaw angle) of the first end effector element (e.g. first end effector element 208-1 shown in Figures 3 to 7) and a second force applied at a second interface element (e.g. interface element 618 shown in Figure 6) for changing an angle (e.g. a yaw angle) of the second end effector element (e.g. second end effector element 208-2 shown in Figures 3 to 7). The first force and the second force may be independently controllable. This may be the case in robotic surgical instruments in which the drive mechanism is capable of independently controlling the yaw rotation of the first and second end effector elements (e.g. as in the robotic surgical instrument 206 shown in Figures 3 to 7). In a specific example, the instrument drive force, $F$, is dependent on a sum of the first force, $f_9$, and the second force, $f_{11}$, - e.g. as defined in Equation 6 above. In this example: each of the first force, $f_9$, and the second force, $f_{11}$, may be specified as negative when their respective end effector element of the robotic surgical instrument is closing, and positive when their respective end effector element of the robotic surgical instrument is opening; or each of the first force, $f_9$, and the second force, $f_{11}$, may be specified as positive when their respective end effector element of the robotic surgical instrument is closing, and negative when their respective end effector element of the robotic surgical instrument is opening. Alternatively, the absolute values of each of the first force, $f_9$, and the second force, $f_{11}$ may be summed.

In a second example, the measured instrument drive force may be dependent on a force applied at a single interface element for changing an angle (e.g. a yaw angle) of one or both of the first end effector element and the second end effector element. This may be the case in robotic surgical instruments in which the first and second end effector elements are not independently rotatable (e.g. yawing of both the first and second end effector elements is driven in tandem by a common pulley, gear, or rack and pinion mechanism), or in which only one of the end effector elements is rotatable whilst the other end effector element remains stationary (e.g. fixed in place relative to the articulation). In these examples, one interface element can control the spread angle between the first and second end effector elements.

It is advantageous to perform the following steps of Figure 19 in dependence on measured instrument drive force data, as described herein, rather than in dependence on data representative of instrument drive forces commanded by the control system. This is because the commanded instrument drive force data, although easy to obtain from the control system logs, may not be fully representative of the instrument drive forces actually applied at the interface elements. For example, there could be faults or losses experienced in the motors being commanded by the control system to generate the instrument drive forces, and/or losses experienced in the drive transfer mechanism between those motors and the interface elements, such that the instrument drive force actually applied at the interface elements differs from the instrument drive force commanded by the control system.

The plurality of measured instrument drive forces may be filtered prior to being used in the following steps of Figure 19 - e.g. so as to mitigate against noisy force sensor readings. The skilled person would be aware of various methods for filtering the readings of a force sensor, and so said filtering methods will not be discussed further herein.

In step 1906, the control system is configured to determine, for each of the plurality of times, a force behaviour value (e.g. force rate value) using a derivative of the measured instrument drive force with respect to the time at that time. For example, the derivative may be the first derivative of the measured instrument drive force with respect to the time. The first derivative of the measured instrument drive force with respect to time at a time is representative of the rate of change of the measured instrument drive force with respect to time at that time. As would be understood by the skilled person, in an example, the first derivative of the measured instrument drive force with respect to time at a time, $t_n$, can be determined by assessing the difference between the measured instrument drive force, $F_n$, at that time, $t_n$, and the measured instrument drive force $F_{n-1}$, at a preceding time, $t_{n-1}$, and dividing that difference, $(F_n - F_{n-1})$, by the time difference between the time $t_n$, and the time $t_{n-1}$, - as represented by Equation 17:

$$\left(\frac{dF}{dt}\right)_n = \left(\frac{F_n - F_{n-1}}{t_n - t_{n-1}}\right) \qquad (17)$$

As would be understood by the skilled person, in other examples, the first derivative of the measured instrument drive force with respect to time at a time, $t_n$, could alternatively be determined by: assessing the difference between the measured instrument drive force, $F_{n+1}$, at a subsequent time, $t_{n+1}$, and the measured instrument drive force $F_n$, at that time, $t_n$, and dividing that difference, $(F_{n+1} - F_n)$, by the time difference between the time $t_{n+1}$, and the time $t_n$; or assessing the difference between the measured instrument drive force, $F_{n+1}$, at a subsequent time, $t_{n+1}$, and the measured instrument drive force $F_{n-1}$, at a preceding time, $t_{n-1}$, and dividing that difference, $(F_{n+1} - F_{n-1})$, by the time difference between the time $t_{n+1}$, and the time $t_{n-1}$.

In step 1908, the control system is configured to determine an offset value in dependence on the plurality of determined force behaviour values.

In the first example (described herein), in step 1908, the offset value may be a spread offset value. In the first example, the control system may be further configured to measure, at each of the plurality of times, a position of each of the one or more interface elements. The positions of each of the one or more interface elements may be measured using a position sensor - such as the position sensor described with reference to Figure 5.

In the first example, the control system may be further configured to predict, for each of the plurality of times, a resultant spread angle between the first end effector element and the second end effector element in dependence on the measured position of each of the one or more interface elements at that time. The control system may use a kinematic equation (e.g. Equation 4 or 5 described herein) to predict the resultant spread angles. In other words, the control system may be configured to predict, for each of the plurality of times, a resultant spread angle between the first end effector element and the second end effector element in dependence on the measured position of each of the one or more interface elements at that time and the geometry of one or more components (e.g. pulleys, gears, rack and pinion mechanism etc.) of a drive transfer mechanism of the robotic surgical instrument configured for transferring force from the one or more interface elements to the first end effector element and/or the second end effector element.

Optionally, the resultant spread angles predicted in the first example of step 1908 may be partially or fully lost motion compensating - as will be described in further detail below with reference to Figure 16.

In the first example, in step 1908, the control system may be further configured to determine the spread offset value in dependence on the plurality of determined force behaviour values and the plurality of predicted resultant spread angles. In particular, in the first example, the control system may be configured to: cause the instrument drive force to be varied to close the robotic surgical instrument from an open configuration, and approximate in dependence on the plurality of determined force behaviour values a time at which the force behaviour value for that time reaches or exceeds a force behaviour threshold value (e.g. $\pm 200$ N/s); and determine, as the spread offset value, the resultant spread angle predicted for that time in dependence on the plurality of predicted resultant spread angles. It is to be understood that the force behaviour threshold may be exceeded and subsequently fallen below one or more times whilst the robotic surgical instrument is being caused to close. For example, this could be owing to mechanical components in the drive transfer mechanism seizing and subsequently releasing - and so the first time the determined force behaviour value exceeds or reaches the force behaviour threshold during closing may not represent the instrument being in a zero configuration. As such, the control system may be configured to: cause the instrument drive force to be varied to close the robotic surgical instrument from an open configuration, and approximate in dependence on the plurality of determined force behaviour values the time at which the force behaviour value for that time first reaches or exceeds the force behaviour threshold value and the plurality of determined force behaviour values do not subsequently fall below the force behaviour threshold whilst the robotic surgical instrument is being caused to close; and determine, as the spread offset value, the resultant spread angle predicted for that time in dependence on the plurality of predicted resultant spread angles. Alternatively, or additionally, the control system may be configured to: cause the instrument drive force to be varied to open the robotic surgical instrument from a closed configuration, and approximate in dependence on the plurality of determined force behaviour values a time at which the force behaviour value for that time reaches or falls below (e.g. first reaches or falls below) a force behaviour threshold value (e.g. $\pm 200$ N/s); and determine, as the spread offset value, the resultant spread angle predicted for that time in dependence on the plurality of predicted resultant spread angles. Suitable force behaviour threshold values may be stored by the control system 124 for each robotic surgical instrument, and/or each robotic surgical instrument may store its suitable force behaviour threshold value in a memory local to that instrument such that that threshold value can be provided to the control system 124 when that instrument is attached to the surgical robot arm.

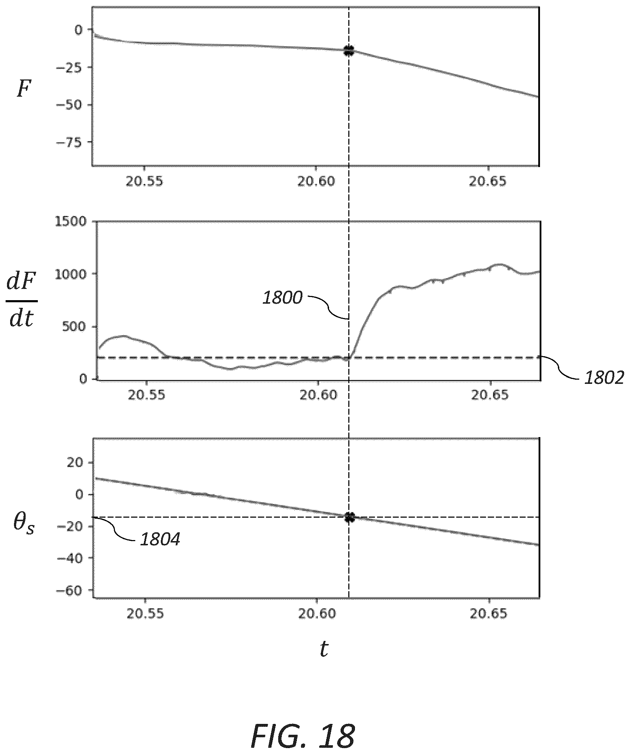

The first example of step 1908 can be understood further with reference to Figure 18 - which illustrates experimental plots (top, middle and bottom) of measured instrument drive force (top), first derivative of measured instrument drive force with respect to time (middle),and predicted resultant spread angle (bottom) against time (top, middle and bottom). Figure 18 illustrates an example in which, in step 1902, the control system is configured to cause the instrument drive force to be varied so as to close the robotic surgical instrument from an open configuration. The determination of a spread offset value in the example illustrated in Figure 18 is described in the following four paragraphs.

By performing steps 1902 and 1904, the control system can generate a plurality of data pairs, each data pair including a measured instrument drive force and a time. A graph can be plotted using these data pairs. The top plot in Figure 18 illustrates an experimental plot of applied instrument drive force, *F,* against time, *t*. In the top plot in Figure 18, the experimental plot can be roughly divided into two portions, each being approximately linear - a first portion on the left-hand-side having a lower gradient, and a second portion on the right-hand-side having a higher gradient. The first portion corresponds to data pairs generated whist the robotic surgical instrument is in an open configuration. The second portion corresponds to data pairs generated whist an inner surface of the first end effector element is exerting a force against an inner surface of the second end effector element - e.g. whilst the robotic surgical instrument is in a closed or "over-closed" configuration. As such, it is desirable to determine where the boundary between the first and second portions lies, as this boundary represents the transition between the open and closed configurations of the robotic surgical instrument. The boundary between the first and second portions can be more easily and accurately identified by differentiating the measured instrument drive force data.

By performing step 1906, the control system can generate a further plurality of data pairs, each data pair including a first derivative of applied instrument drive force with respect to time, $\frac{dF}{dt}$, and a time, *t*. A graph can be plotted using these data pairs. The middle plot in Figure 18 illustrates an experimental plot of the first derivative of applied instrument drive force with respect to time, $\frac{dF}{dt}$, against time, *t*. The data shown in the middle plot in Figure 18 has been derived by determining force behaviour values for the data shown in the top plot in Figure 18.

By predicting, for each of the plurality of times, a resultant spread angle between the first end effector element and the second end effector element in dependence on the measured position of each of the one or more interface elements at that time, the control system can generate another plurality of data pairs, each data pair including a predicted resultant spread angle, $\theta_s$, and a time, *t*. A graph can be plotted using these data pairs. The bottom plot in Figure 18 illustrates an experimental plot of predicted resultant spread angle, $\theta_s$, against time, *t*. The data shown in the bottom plot in Figure 18 has been derived for the same plurality of times as the data shown in the top and middle plots in Figure 18.

The determination of a spread offset value in the example illustrated in Figure 18 may comprise approximating, in dependence on the plurality of determined force behaviour values (e.g. with reference to the middle plot of Figure 18), a time 1800 at which the force behaviour value for that time reaches or exceeds (e.g. first reaches or exceeds, and the plurality of determined force behaviour values do not subsequently fall below) a force behaviour threshold value 1802; and determining, as the spread offset value, the resultant spread angle 1804 predicted for that time 1800 in dependence on the plurality of predicted resultant spread angles (e.g. with reference to the bottom plot of Figure 18). In the specific example shown in Figure 18, the spread offset value is -13.6°. This means that, in this Figure 18 example, the control system has determined that the robotic surgical instrument was in a "zero configuration", as defined herein, when the kinematic equations were predicting a spread angle of - 13.6° (i.e. an over-closed configuration). It follows that, when the kinematic equations were predicting a spread angle of 0°, the robotic surgical instrument was in fact in an open configuration (e.g. with a spread angle of approximately +13.6°).

In the second example (described herein), in step 1908, the offset value may be an interface position offset value. As described herein, in the second example, the drive mechanism may comprise an interface element (e.g. a single interface element, or only one interface element) that can control the spread angle between the first and second end effector elements - e.g. when a robotic surgical instrument in which the first and second end effector elements are not independently rotatable (e.g. yawing of both the first and second end effector elements is driven in tandem by a common pulley, gear, or rack and pinion mechanism), or in which only one of the end effector elements is rotatable whilst the other end effector element remains stationary (e.g. fixed in place relative to the articulation), is attached to the surgical robot arm.

In the second example, the control system may be further configured to measure, at each of the plurality of times, a position of an interface element (e.g. a single interface element, or only one interface element) of the one or more interface elements that is indicative of a predicted spread angle between the first end effector element and the second end effector element. The positions of the interface element may be measured using a position sensor - such as the position sensor described with reference to Figure 5.

Optionally, the positions measured in the second example of step 1908 may be partially or fully lost motion compensating - as will be described in further detail below with reference to Figure 16.

In the second example, in step 1908, the control system may be further configured to determine the interface position offset value in dependence on the plurality of determined force behaviour values and the plurality of measured interface positions. In particular, in the second example, the control system may be configured to: cause the instrument drive force to be varied to close the robotic surgical instrument from an open configuration, and approximate in dependence on the plurality of determined force behaviour values a time at which the force behaviour value for that time reaches or exceeds a force behaviour threshold value (e.g. ±200 N/s); and determine, as the interface position offset value, the interface position at that time in dependence on the plurality of measured interface positions. It is to be understood that the force behaviour threshold may be exceeded and subsequently fallen below one or more times whilst the robotic surgical instrument is being caused to close. For example, this could be owing to mechanical components in the drive transfer mechanism seizing and subsequently releasing - and so the first time the determined force behaviour value exceeds or reaches the force behaviour threshold during closing may not represent the instrument being in a zero configuration. As such, the control system may be configured to: cause the instrument drive force to be varied to close the robotic surgical instrument from an open configuration, and approximate in dependence on the plurality of determined force behaviour values the time at which the force behaviour value for that time first reaches or exceeds the force behaviour threshold value and the plurality of determined force behaviour values do not subsequently fall below the force behaviour threshold whilst the robotic surgical instrument is being caused to close; and determine, as the interface position offset value, the interface position at that time in dependence on the plurality of measured interface positions.. Alternatively, or additionally, the control system may be configured to: cause the instrument drive force to be varied to open the robotic surgical instrument from a closed configuration, and approximate in dependence on the plurality of determined force behaviour values a time at which the force behaviour value for that time reaches or falls below (e.g. first reaches or falls below) a force behaviour threshold value (e.g. ±200 N/s); and determine, as the interface position offset value, the interface position at that time in dependence on the plurality of measured interface positions. Suitable force behaviour threshold values may be stored by the control system 124 for each robotic surgical instrument and/or each robotic surgical instrument may store its suitable force behaviour threshold value in a memory local to that instrument such that that threshold can be provided to the control system 124 when that instrument is attached to the surgical robot arm.

In both the first and second examples, it is advantageous to perform step 1908 of Figure 19 by approximating when a first derivative of the measured instrument drive force with respect to time crosses (e.g. exceeds or falls below) a threshold. For example, step 1908 could alternatively be performed by approximating when a second derivative of the measured instrument drive force with respect to time reaches a minimum or maximum. Assuming that a minimum or maximum could be correctly identified, this alternative approach may even be capable of more accurately identifying the precise time when a transition between an open and closed configuration of the robotic surgical instrument occurred. That said, it can be very difficult to correctly identify a minimum or maximum in the second derivative of the measured instrument drive force with respect to time, especially in examples where the control system has caused the instrument drive force to be varied so as to open the robotic surgical instrument from a closed configuration. For example, it may be difficult for the control system to correctly distinguish between local and global minimum or maximum. By contrast, it is straightforward to correctly identify when a first derivative of the measured instrument drive force with respect to time crosses a threshold - whether or not the instrument drive force has been varied so as to open the robotic surgical instrument from a closed configuration, or close the robotic surgical instrument from an open configuration. As such, using this "first derivative threshold" approach, the control system can more reliably identify a time when a transition between an open and closed configuration of the robotic surgical instrument occurred. The inventors have found that, on balance, the greatly increased reliability of the "first derivative threshold" approach outweighs the slightly increased accuracy of the "second derivative minimum/maximum" approach.

In order to mitigate against the control system performing steps 1906 and 1908 whilst the robotic surgical instrument is grasping an object between its end effector elements (e.g. whilst robotic surgical instrument the in an open configuration), the control system may be configured to perform steps 1906 and 1908 in response to determining that: a plurality of predicted resultant spread angles are within a predetermined range of spread angles (e.g. between 20° and -20°); a plurality of measured instrument drive forces within a predetermined range of instrument drive forces (e.g. between -10N and -50N); the predicted resultant spread angles are changing at a rate faster than a predetermined threshold (e.g. - 30°/s, which represents robotic surgical instrument closing at a rate of 30°/s); a plurality of measured positions are within a predetermined range of measured positions (e.g. a predetermined range of measured positions that are indicative of predicted spread angles of between -20° and +20° - e.g., for an example robotic surgical instrument, the predetermined range of measured positions may be -2mm to +2mm); and/or the measured positions are changing at a rate faster than a predetermined threshold (e.g. a predetermined threshold rate indicative of the robotic surgical instrument closing at a rate of 30°/s). Suitable thresholds and/or ranges may be stored by the control system 124 for each robotic surgical instrument and/or each robotic surgical instrument may store its suitable thresholds and/or ranges in a memory local to that instrument such that those thresholds and/or ranges can be provided to the control system 124 when that instrument is attached to the surgical robot arm.

In step 1910, the control system is configured to control, in dependence on the offset value, the surgical robot arm to control the spread angle between the first end effector element and the second end effector element of the robotic surgical instrument. In an example, the control system can be configured to receive a request to drive the spread angle between the first end effector element and the second end effector element to a desired spread angle. For example, an input may be received at the surgeon input device, that input being indicative of a request to drive the spread angle to a desired spread angle. The control system can be configured to determine a commanded spread angle in dependence on the desired spread angle and the offset value. The control system can be configured to control the surgical robot arm to drive the robotic surgical instrument using the commanded spread angle.

In the first example, in step 1910, the control system can be configured to control the surgical robot arm to drive the robotic surgical instrument in dependence on the spread offset value. The control system may be configured to use the spread offset value to adjust subsequently predicted resultant spread angles. For example, the control system may be configured to subtract the spread offset value from the output of the kinematic equations used to predict a resultant spread angle for an applied instrument drive force so as to determine a calibrated spread angle. In this way, the control system can control the surgical robot arm such that an appropriate instrument drive force is applied in order that the robotic surgical instrument is driven to a configuration (e.g. a spread angle) that more closely matches the configuration (e.g. the spread angle) requested by the input received at the surgeon input device. In a specific example, the control system may be configured to use the spread offset value to determine a calibrated zero configuration for the robotic surgical instrument. In this way, the control system can control the surgical robot arm such that an appropriate instrument drive force is applied in order that the robotic surgical instrument is driven to the calibrated zero configuration when the input received at the surgeon input device requests that the robotic surgical instrument is in the zero configuration. For example, referring to the example shown in Figure 18, in response to receiving an input at the surgeon input device requesting that the robotic surgical instrument is in the zero configuration (e.g. having a spread angle of 0°), the control system may control the surgical robot arm to apply an appropriate instrument drive force to drive the robotic surgical instrument to a predicted spread angle of -13.6° (e.g. as subtracting the spread offset value, -13.6°, from the predicted spread angle, -13.6°, gives an adjusted spread angle of 0°). That is, in this example, the control system can cause the one or more interface elements to be driven to respective measured positions that, when input to the kinematic equations, output a predicted spread angle of -13.6°. In another example, the control system can be configured to receive a request to drive the spread angle between the first end effector element and the second end effector element to a desired spread angle (e.g. 20°, in an illustrative example). The control system can be configured to determine a commanded spread angle in dependence on the desired spread angle (e.g. 20°) and the offset value (e.g. -13.6°). Determining the commanded spread angle in dependence on the desired spread angle and the offset value may comprise summing the desired spread angle (e.g. 20°) and the offset value (e.g. -13.6°) so as to output a commanded spread angle (e.g. 6.4°, in the illustrative example). The control system can be configured to control the surgical robot arm to drive the robotic surgical instrument using the commanded spread angle. That is, in this illustrative example, in order to cause the spread angle to be driven to the desired 20°, the control system may command the robot arm to drive the spread angle to a predicted spread angle of 6.4°.

In the second example, in step 1910, the control system can be configured to control the surgical robot arm to drive the robotic surgical instrument in dependence on the interface position offset value. The control system may be configured to use the interface position offset value to adjust subsequently measured interface positions that are to be input to the kinematic equations used to predict a resultant spread angle for an applied instrument drive force. For example, the control system may be configured to subtract the interface position offset value from the measured interface positions so as to determine calibrated interface positions to be input to the kinematic equations. In this way, the control system can control the surgical robot arm such that an appropriate instrument drive force is applied in order that the robotic surgical instrument is driven to a configuration (e.g. a spread angle) that more closely matches the configuration (e.g. the spread angle) requested by the input received at the surgeon input device. In a specific example, the control system may be configured to use the interface position offset value to determine a calibrated zero configuration for the robotic surgical instrument. In this way, the control system can control the surgical robot arm such that an appropriate instrument drive force is applied in order that the robotic surgical instrument is driven to the calibrated zero configuration when the input received at the surgeon input device requests that the robotic surgical instrument is in the zero configuration.

Configuring the control system (e.g. control system 124 shown in Figure 1) to perform steps 1902, 1904, 1906, 1908 and 1910 can improve the surgeon's perception that the movements of the robotic surgical instrument are accurately reflecting the inputs they provide to the surgeon input device.

The control system can be configured to perform steps 1902, 1904, 1906 and 1908 as part of a calibration process prior to using a robotic surgical instrument to perform aspects of a minimally invasive surgical procedure. For example, such a calibration process may be performed prior to beginning a minimally invasive surgical procedure, and/or each time a new robotic surgical instrument is attached to the surgical robot arm during the minimally invasive surgical procedure.

Alternatively, or additionally, the control system can be configured to perform steps 1902, 1904, 1906 and 1908 iteratively during a minimally invasive surgical procedure, so as to iteratively update the determined offset value. For example, the control system may be configured to automatically perform steps 1906 and 1908 each time the conditions for determining a force behaviour value, as described herein with reference to step 1906 and 1908, are met during a minimally invasive surgical procedure. This approach of iteratively updating the determined offset value can advantageously take account of "wear and tear" of the robotic surgical instrument during the minimally invasive surgical procedure. In these examples, in order to mitigate against the control system determining an offset value whilst the robotic surgical instrument is grasping an object between its end effector elements (e.g. whilst robotic surgical instrument the in an open configuration) the control system may be configured to determine the offset value in dependence on a range of permissible spread offset values. In the first example, the range of permissible spread offset values may be -15° to +15°. The spread offset value may not be updated if the control system determines a spread offset value outside of that permissible range. In the second example, the range of permissible interface position offset values may be indicative of predicted spread angles of -15° to +15° - e.g., for an example robotic surgical instrument, the range of permissible interface position offset values may be -1.2mm to +1.2mm as defined relative to the neutral (e.g. resting) position of the interface element, and/or -10% to +10% of the total possible travel of an interface element relative to its neutral (e.g. resting) position (e.g. at 50%, but not necessarily). The interface position offset value may not be updated if the control system determines an interface position offset value outside of that permissible range.

As described herein, in step 1902, the control system may be configured to cause the instrument drive force to be varied such that the robotic surgical instrument performs a "calibration routine". In some examples, the control system may be configured to perform steps 1904 to 1908 more than once during such a calibration routine. That is, the control system may be configured to determine more than one offset value. For example, the control system may determine a first offset value from data obtained when closing the robotic surgical instrument from an open configuration to a closed configuration, and determine a second offset value from data obtained when opening the robotic surgical instrument from a closed configuration to an open configuration. The first offset value and the second offset value may not be exactly the same - e.g. owing to being determined from data obtained when driving the robotic surgical instrument in different directions. In another example, the control system may determine a first offset value from data obtained when closing the robotic surgical instrument from an open configuration to a closed configuration for a first time, and determine a second offset value from data obtained when closing the robotic surgical instrument from an open configuration to a closed configuration for a second time. The first offset value and the second offset may not be exactly the same - e.g. owing to unavoidable variability, error and/or noise in the data. The control system may be configured to combine multiple offset values (e.g. as determined from data obtained during a calibration routine). For example, the control system may be configured to determine a combined offset value by averaging multiple offset values, or by combining multiple offset values using a weighted sum (e.g. where offset values determined from data obtained when closing the instrument are assigned a greater weight than offset values determined from data obtained when opening the instrument). The offset values that are combined are the same type of offset values - e.g. multiple spread offset values as determined in the first example described herein, or multiple interface position offset values as determined in the second example described herein. In step 1910, the control system may be configured to control the surgical robot arm in dependence on the combined offset value. In other words, the control system may be configured to: determine an offset value in dependence on the plurality of determined force behaviour values; determine a further offset value in dependence on the plurality of determined force behaviour values; combine the offset value and the further offset value to form a combined offset value; and control, in dependence on the combined offset value, the surgical robot arm to control the spread angle between the first end effector element and the second end effector element of the robotic surgical instrument.

In some examples, the control system may be configured to perform the set of steps described herein with reference to Figure 19 in combination with any one or more of the sets of steps described herein with reference to Figures 9, 10, 13 and/or 14.

In the first example described herein, the control system may be configured to perform the set of steps described herein with reference to Figure 19 in order to determine a first spread offset value; and perform the set of steps described herein with reference to Figure 9 to determine a second spread offset value; and/or perform the set of steps described herein with reference to Figure 13 to determine a third spread offset value. That is, in addition to the set of steps described herein with reference to Figure 19, the control system may be further configured to: fit a linear relationship to at least two measured instrument drive force and predicted resultant spread angle data pairs, wherein a data pair comprises an instrument drive force measured at a time of the plurality of times and a resultant spread angle predicted for the same time of the plurality of times; and determine a second spread offset value, wherein determining the second spread offset value comprises approximating, using the linear relationship, a predicted spread angle when the measured instrument drive force would equal a predetermined force value - as described herein with reference to Figure 9. Alternatively, or additionally, in addition to the set of steps described herein with reference to Figure 19, the control system may be further configured to: determine, for each of the plurality of predicted resultant spread angles, an instrument stiffness value using a derivative of the measured instrument drive force with respect to the predicted resultant spread angle at that predicted resultant spread angle; and determine a third spread offset value in dependence on the plurality of determined instrument stiffness values - e.g. as described herein with reference to Figure 13. The first, second and third spread offset values may not be exactly the same - e.g. owing to being determined using different techniques. The control system may be configured to combine the first spread offset value with the second spread offset value and/or the third spread offset value in order to form a combined spread offset value. For example, the control system may be configured to form a combined spread offset value by averaging the first, second and/or third spread offset values, or by combining the first, second and/or third spread offset values using a weighted sum (e.g. where a different weighting is assigned to the spread offset value determined using each technique, e.g. where the technique deemed to determine the most accurate spread offset values is associated with the greatest weighting). In step 1910, the control system may be configured to control the surgical robot arm in dependence on the combined spread offset value.

In the second example described herein, the control system may be configured to perform the set of steps described herein with reference to Figure 19 in order to determine a first interface position offset value; and perform the set of steps described herein with reference to Figure 10 to determine a second interface position offset value; and/or perform the set of steps described herein with reference to Figure 14 to determine a third interface position offset value. That is, in addition to the set of steps described herein with reference to Figure 19, the control system may be further configured to: fit a linear relationship to at least two measured instrument drive force and measured interface position data pairs, wherein a data pair comprises an instrument drive force measured at a time of the plurality of times and an interface position measured at the same time of the plurality of times; and determine a second interface position offset value, wherein determining the second interface position offset value comprises approximating, using the linear relationship, an interface position when the measured instrument drive force would equal a predetermined force value - e.g. as described herein with reference to Figure 10.

Alternatively, or additionally, in addition to the set of steps described herein with reference to Figure 19, the control system may be further configured to: determine, for each of the plurality of measured interface positions, an instrument stiffness value using a derivative of the measured instrument drive force with respect to the measured interface position at that measured interface position; and determine a third interface position offset value in dependence on the plurality of determined instrument stiffness values - e.g. as described herein with reference to Figure 14. The first, second and third interface position offset values may not be exactly the same - e.g. owing to being determined using different techniques. The control system may be configured to combine the first interface position offset value with the second interface position offset value and/or the third interface position offset value in order to form a combined interface position offset value. For example, the control system may be configured to form a combined interface position offset value by averaging the first, second and/or third interface position offset values, or by combining the first, second and/or third interface position offset values using a weighted sum (e.g. where a different weighting is assigned to the interface position offset value determined using each technique, e.g. where the technique deemed to determine the most accurate interface position offset values is associated with the greatest weighting). In step 1910, the control system may be configured to control the surgical robot arm in dependence on the combined interface position offset value.

The control system may be configured to perform any one or more of the sets of steps described herein with reference to Figures 9, 10, 13, 14 and 19. The control system may be configured to perform a different one of the sets of steps described herein with reference to Figures 9, 10, 13, 14 and 19 depending on which (or what type of) robotic surgical instrument is attached to the surgical robot arm.

## *Lost motion compensation*

Optionally, the control system can be configured to control the surgical robot arm to drive the robotic surgical instrument to compensate for lost motion within the drive mechanism and/or the robotic surgical instrument. The control system can be configured to perform said lost motion compensation in combination with any of the sets of steps described herein with reference to Figures 9, 10, 13, 14 and 19 - although this need not be the case. Configuring the control system in this way can advantageously further improve the surgeon's perception that the movements of the robotic surgical instrument are accurately reflecting the inputs they provide to the surgeon input device.

Lost motion, sometimes referred to as backlash, can be caused by the attachment between the drive mechanism of the surgical robot arm and the robotic surgical instrument, and/or one or more components in the drive transfer mechanism of the robotic surgical instrument. For example, lost motion can be caused by one or more cables in the drive transfer mechanism of certain robotic surgical instruments stretching. Another source of lost motion can be the sterile barrier (e.g. sheet of flexible material) positioned between the drive mechanism and the robotic surgical instrument. Lost motion can cause a time lag between an interface element in the drive mechanism moving, and the corresponding end effector element(s) of the robotic surgical instrument moving.

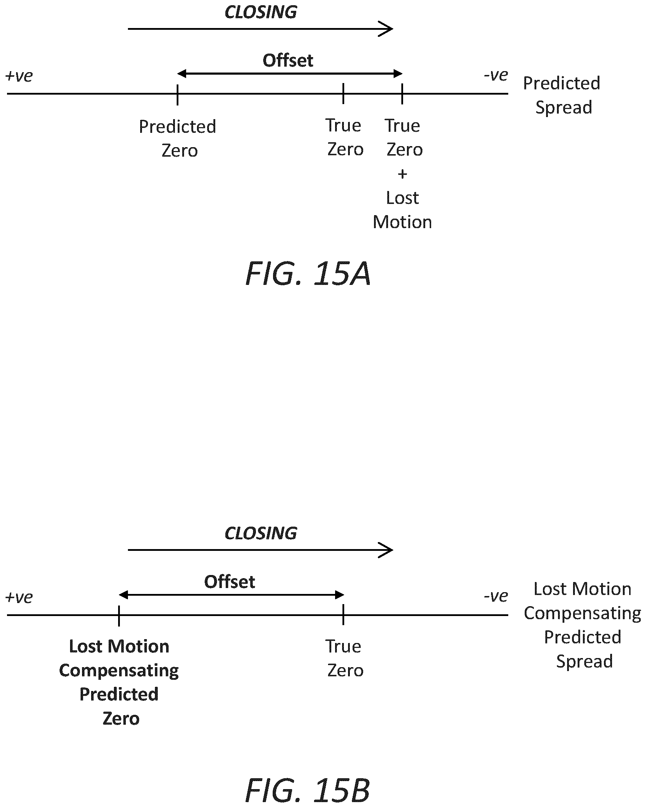

The effects of lost motion can be understood with reference to Figure 15A - which illustrates the true zero configuration and spread offset value relative to predicted resultant spread angles when closing a robotic surgical instrument. In Figure 15A, the axis 1500 represents the predicted resultant spread angles for a robotic surgical instrument. As described herein, said predicted resultant spread angles can be determined using kinematic equations, such Equations 4 or 5. As shown by arrow 1502, Figure 15A is considering an example in which the robotic surgical instrument is closing from an open configuration. That is, the robotic surgical instrument is closing from a positive (i.e. "+ve") predicted spread angle to a negative (i.e. "-ve") predicted spread angle. The predicted spread angle (i.e. predicted 0°) at which the kinematic equations predict the robotic surgical instrument will be in the zero configuration is shown as "predicted zero" 1504. The predicted spread angle at which the robotic surgical instrument is actually in the zero configuration is shown as "true zero" 1506. Using the sets of steps described herein with reference to Figures 9 or 13, a spread offset value 1508 can be determined. However, as shown in Figure 15A, owing to the effects of lost motion, the spread offset value 1508 can be larger than the actual offset between the "predicted zero" 1504 and the "true zero" 1506. This is because the sets of steps described herein with reference to Figures 9 or 13 use sensed instrument drive force and measured interface element position data collected at the drive mechanism, and so do not fully account for lost motion occurring "downstream" of that drive mechanism (e.g. in the sterile barrier and/or in the drive transfer mechanism of the surgical robot instrument).

The amount of difference between the "true zero" 1504" and "true zero plus lost motion" 1510, i.e. the amount of lost motion, can depend on the drive transfer mechanism of the robotic surgical instrument. For example, a significant source of lost motion is cable stretch. Hence, a drive transfer mechanism that uses cables with a lower resistance to elastic deformation (e.g. having a lower Young's modulus) would experience more lost motion relative to a drive transfer mechanism that uses cables with a higher resistance to elastic deformation (e.g. having a higher Young's modulus). Drive transfer mechanisms that use push-rods to transfer drive forces rather than cables may experience little to no lost motion - meaning that the lost motion compensation techniques described herein need not be performed for robotic surgical instruments having these types of drive transfer mechanisms.

*Approach 1 - determining a lost motion compensating interface element position*

Figure 16 illustrates a first set of steps performed by the control system for compensating for lost motion. The control system can be configured to perform the sets of steps described herein with reference to Figure 16 in combination with any of the sets of steps described herein with reference to Figures 9, 10, 13, 14 and 19 - although this need not be the case.

In step 1602, the control system is configured to cause an instrument drive force applied at the one or more interface elements to be varied. The applied forces may be measured using force sensors - such as those described with reference to Figure 5. In step 1602, the control system may be configured to cause the instrument drive force to be varied so as to close the robotic surgical instrument from an open configuration.

In step 1604, the control system is configured to determine one or more interface stiffness values for each of the one or more interface elements. Each of the interface stiffness values is based on a derivative of the force applied at that interface element with respect to the measured position of that interface element. For example, Equation 11 can be used to determine an interface stiffness value for each of a first interface element (e.g. interface element 616 shown in Figure 6) for changing an angle (e.g. a yaw angle) of the first end effector element (e.g. first end effector element 208-1 shown in Figures 3 to 7) and a second interface element (e.g. interface element 618 shown in Figure 6) for changing an angle (e.g. a yaw angle) of the second end effector element (e.g. second end effector element 208-2 shown in Figures 3 to 7). In Equation 11, $f_9$ is the force applied at the first interface element, $q_9$ is the measured position of the first interface element, $k_9$ is an interface stiffness value for the first interface element, $f_{11}$ is the force applied at the second interface element, $q_{11}$ is the measured position of the second interface element, and $k_{11}$ is an interface stiffness value for the second interface element.

$$\frac{df_{9,11}}{dq_{9,11}} = k_{9,11} \qquad (11)$$

The control system may be configured to determine one or more interface stiffness values for each of the one or more interface elements in response to determining that: the predicted resultant spread angle for the applied instrument drive force is within a predetermined range of spread angles (e.g. between 20° and -20°); and/or the predicted resultant spread angles for a series of applied instrument drive forces are changing at a rate faster than a predetermined threshold (e.g. -30°/s, which represents robotic surgical instrument closing at a rate of 30°/s). Suitable thresholds and/or ranges may be stored by the control system 124 for each robotic surgical instrument and/or each robotic surgical instrument may store its suitable thresholds and/or ranges in a memory local to that instrument such that those thresholds and/or ranges can be provided to the control system 124 when that instrument is attached to the surgical robot arm.

The control system can be configured to determine a plurality of interface stiffness values for each of the one or more interface elements. For example, a plurality of interface stiffness values can be calculated at a respective plurality of different applied instrument drive forces. The control system can be configured to determine an average interface stiffness value for each of the one or more interface elements in dependence on the plurality of interface stiffness values. For example, Equation 12 determines an average interface stiffness value for each of the first the second interface elements - i.e. average interface stiffness values $\overline{k_9}$ and $\overline{k_{11}}$, respectively. In Equation 12, each of the average interface stiffness

values $\overline{k_9}$ and $\overline{k_{11}}$ can also be multiplied by a correction factor $\alpha$. A suitable value for the correction factor $\alpha$ can be determined empirically. A suitable correction factor $\alpha$ may be stored by the control system 124 for each robotic surgical instrument, and/or each robotic surgical instrument may store its suitable correction factor $\alpha$ in a memory local to that instrument such that that correction factor can be provided to the control system 124 when that instrument is attached to the surgical robot arm. By way of example, a correction factor $\alpha$ of 0.015 was found to work well for the example robotic surgical instrument and drive mechanism shown in Figures 3 to 6. The output of Equation 12 is a corrected average interface stiffness value for each of the first the second interface elements - i.e. corrected average interface stiffness values $k'_9$ and $k'_{11}$, respectively.

$$k'_{9,11} = \alpha \overline{k_{9,11}} \qquad (12)$$

For each of the one or more interface elements, in step 1606, the control system is configured to determine, for an instrument drive force applied at that interface element, a lost motion compensating position of that interface element in dependence on the one or more determined interface stiffness values (e.g. the corrected average interface stiffness). For example, Equation 13 determines a lost motion compensating position for each of the first the second interface elements - i.e. lost motion compensating position $\tilde{q}_9$ and $\tilde{q}_{11}$, respectively. Optionally, the sensed applied instrument drive forces, $f_{9,11}$, can be filtered prior to being input to Equation 13 so as to mitigate against noisy force sensor readings. The skilled person would be aware of various methods for filtering the readings of a force sensor, and so said filtering methods will not be discussed further herein.

$$\tilde{q}_{9,11} = q_{9,11} - \frac{f_{9,11}}{k'_{9,11}} \qquad (13)$$

The lost motion compensating position of an interface element is a theoretical or hypothetical position of that interface element that is intended to compensate for the effects of lost motion when used in kinematic equations to predict the resultant spread angle of the robotic surgical instrument at that applied instrument drive force.

In step 1608, the control system is configured to predict, for the instrument drive force applied by the drive mechanism, a lost motion compensating resultant spread angle in dependence on the determined lost motion compensating positions of each of the one or more interface elements. For example, Equation 14 (based on Equation 5 described herein) predicts a lost motion compensating resultant spread angle, $\tilde{\theta}_s$, using the lost motion compensating positions $\tilde{q}_9$ and $\tilde{q}_{11}$.

$$\tilde{\theta}_s = \frac{\tilde{q}_{11}}{r_{y1}} + \frac{\tilde{q}_9}{r_{y2}} \qquad (14)$$

The advantages of configuring the control system to predict lost motion compensating resultant spread angles as described herein with reference to Figure 16 can be understood with reference to Figure 15B - which illustrates the true zero configuration and spread offset value relative to lost motion compensating predicted resultant spread angles when closing a robotic surgical instrument. Figure 15B uses the same raw applied instrument drive force and interface element position data as Figure 15A. In Figure 15B, the axis 1520 represents the lost motion compensating predicted resultant spread angles for a robotic surgical instrument. As shown by arrow 1522, Figure 15B is considering an example in which the robotic surgical instrument is closing from an open configuration. That is, the robotic surgical instrument is closing from a positive (i.e. "+ve") lost motion compensating predicted spread angle to a negative (i.e. "-ve") lost motion compensating predicted spread angle. The lost motion compensating predicted spread angle (i.e. lost motion compensating predicted 0°) at which the lost motion compensating kinematic equations predict the robotic surgical instrument will be in the zero configuration is shown as "lost motion compensating predicted zero" 1524. The predicted spread angle at which the robotic surgical instrument is actually in the zero configuration is shown as "true zero" 1506. Using the sets of steps described herein with reference to Figures 9 or 13, a spread offset value 1508 can be determined. In Figure 15B (unlike in Figure 15A), the spread offset value 1508 matches the actual offset between the "lost motion compensating predicted zero" 1524 and the "true zero" 1506.

As a result, a combination of the lost motion compensating resultant spread angles predicted as described herein with reference to Figure 16 and the spread offset value determined as described herein with reference to Figures 9 or 13, can be used to determine calibrated spread angles for the robotic surgical instrument.

The sets of steps described herein with reference to Figure 16 to predict a lost motion compensating resultant spread angle, $\tilde{\theta}_s$, can be performed in parallel with the sets of steps described herein with reference to Figures 9 or 13 to determine a spread offset value. This is shown in Figure 17, which illustrates a set of steps performed by the control system for determining a calibrated spread angle.

In step 1702, the control system is configured to cause an instrument drive force applied at the one or more interface elements to be varied as described herein with reference to steps 902, 1002, 1302, 1402 and/or 1602.

In step 1704, the control system is configured to predict, for an instrument drive force applied by the drive mechanism, a lost motion compensating resultant spread angle, $\tilde{\theta}_S$, as described herein with reference to Figure 16.

In step 1706, the control system is configured to determine a spread offset value, $S_0$, as described herein with reference to Figures 9 or 13.

In step 1708, the control system is configured to determine, for the instrument drive force applied by the drive mechanism, a calibrated spread angle, $\theta'_S$, in dependence on the predicted lost motion compensating resultant spread angle, $\tilde{\theta}_S$, and the spread offset value, $S_0$. For example, the control system may be configured to subtract the spread offset value, $S_0$, from the predicted lost motion compensating resultant spread angle, $\tilde{\theta}_S$, so as to determine a calibrated spread angle, $\theta'_S$, for that instrument drive force applied by the drive mechanism (e.g. see Equation 15).

$$\theta'_S = \tilde{\theta}_S - S_0 \qquad (15)$$

The calibrated spread angle, $\theta'_S$, is a more accurate estimate of the actual spread angle between the first end effector element and the second end effector element when a given instrument drive force is applied. By determining calibrated spread angles, the control system can control the surgical robot arm such that an appropriate instrument drive force is applied in order that the robotic surgical instrument is driven to a configuration (e.g. a calibrated spread angle) that more closely matches the configuration (e.g. the spread angle) requested by the input received at the surgeon input device. That is, the control system can cause the one or more interface elements to be driven to respective measured positions that, when input to the kinematic equations, output the calibrated spread angle.

It is to be understood that, in examples where a single interface element can control the spread angle between the first and second end effector elements, e.g. as discussed with reference to Figures 10 and 14, the lost motion compensating position of that interface element output from step 1606 can be used to determine a calibrating position of the interface element. That is, in these examples step 1608 need not be performed. Instead, in these examples, the control system can be configured to determine, for an instrument drive force applied by the drive mechanism, a calibrating position of the interface element in dependence on the predicted lost motion compensating position of the interface element output from step 1606 and an interface position offset value, $q_0$, determined as described with reference to Figures 10 and 14. In an example, the control system may be configured to subtract the interface position offset value, $q_0$, from the predicted lost motion compensating position, $\tilde{q}$, of the interface element so as to determine a calibrating position, $q'$, of the interface element for that instrument drive force applied by the drive mechanism (e.g. see Equation 16).

$$q' = \tilde{q} - q_0 \qquad (16)$$

The control system may be configured to input the calibrating position, $q'$, into the kinematic equations used to predict a resultant spread angle for an applied instrument drive force so as to output the calibrated spread angle. In this way, the control system can control the surgical robot arm such that an appropriate instrument drive force is applied in order that the robotic surgical instrument is driven to a configuration (e.g. a calibrated spread angle) that more closely matches the configuration (e.g. the spread angle) requested by the input received at the surgeon input device.

It is to be understood that the sets of steps described herein with reference to Figure 16 could be performed independently of (i.e. need not necessarily be performed in combination with) the sets of steps described herein with reference to Figures 9, 10, 13, 14 and 19.

*Approach 2 - approximating a lost motion compensation value*

Figure 20 illustrates a second set of steps performed by the control system for controlling a robotic surgical instrument in dependence on an approximated lost motion compensation value. The set of steps described herein with reference to Figure 20 is a computationally less expensive alternative to the sets of steps described herein with reference to Figure 16. The control system can be configured to perform the set of steps described herein with reference to Figure 20 in combination with any of the sets of steps described herein with reference to Figures 9, 10, 13, 14 and 19 - although this need not be the case.

In step 2002, the control system is configured to receive a request to drive the spread angle between the first end effector element and the second end effector element to a desired spread angle. For example, an input may be received at the surgeon input device, that input being indicative of a request to drive the spread angle to a desired spread angle. The input may be indicative of a request to increase the spread angle from the current spread angle to the desired spread angle. The input may be indicative of a request to decrease the spread angle from the current spread angle to the desired spread angle. The control system may be configured to interpret inputs received at the surgeon input device as indicating requests for desired spread angles for the robotic surgical instrument at a predetermined frequency, e.g. of 2.5 kHz. As such, steps 2002, 2004 and 2006 can be repeated at the predetermined frequency, e.g. a frequency of 2.5kHz, so as to control the spread angle between the first end effector element and the second end effector element.

In step 2004, the control system is configured to approximate a lost motion compensation value in dependence on a lost motion compensation function. Approximating a lost motion compensation value in dependence on the lost motion compensation function may comprise modifying (e.g. translating, e.g. horizontally translating) the lost motion compensation function when the direction in which the first and second end effector elements are to be driven in dependence on the desired spread angle is changed (e.g. from opening to closing, or vice versa) - as will be described in further detail herein.

In a first example, the lost motion compensation function relates desired spread angle to lost motion compensation value. That is, in the first example, the desired spread angle can be input into the lost motion compensation function. In a second example, the lost motion compensation function relates offset-corrected spread angle to lost motion compensation value. That is, in the second example, the offset value determined using any of the sets of steps described herein with reference to Figures 9, 10, 13, 14 and 19 can be used to adjust the desired spread angle (e.g. by summing the desired spread angle and the offset value) in order to form an offset-corrected spread angle, and the offset-corrected spread angle can be input into the lost motion compensation function.

The lost motion compensation function may be used to approximate a lost motion spread compensation value. For example, a lost motion spread compensation value may represent an additional amount (e.g. positive or negative) of spread angle change that is to be commanded by the control system when driving the robotic surgical instrument in response to a request to drive the spread angle to a desired spread angle. Alternatively, in examples where a single interface element can control the spread angle between the first and second end effector elements, e.g. as discussed with reference to Figures 10 and 14, the lost motion compensation function may be used to approximate a lost motion interface position compensation value. For example, a lost motion interface position compensation value may represent an additional amount (e.g. positive or negative) of interface position change that is to be commanded by the control system when driving the robotic surgical instrument in response to a request to drive the spread angle to a desired spread angle.

The lost motion compensation function may comprise a first portion that relates desired or offset-corrected spread angle to lost motion compensation value for a range of desired or offset-corrected spread angles. The lost motion compensation function may also comprise a second portion that is representative of a maximum positive lost motion compensation value. The first portion may connect to the second portion at a first transition point. The first transition point may be at the greatest desired or offset-corrected spread angle within the range of desired or offset-corrected spread angles. The lost motion compensation function may also comprise a third portion that is representative of a maximum negative lost motion compensation value. The first portion may connect to the third portion at a second transition point. The second transition point may be at the smallest desired or offset-corrected spread angle within the range of desired or offset-corrected spread angles.

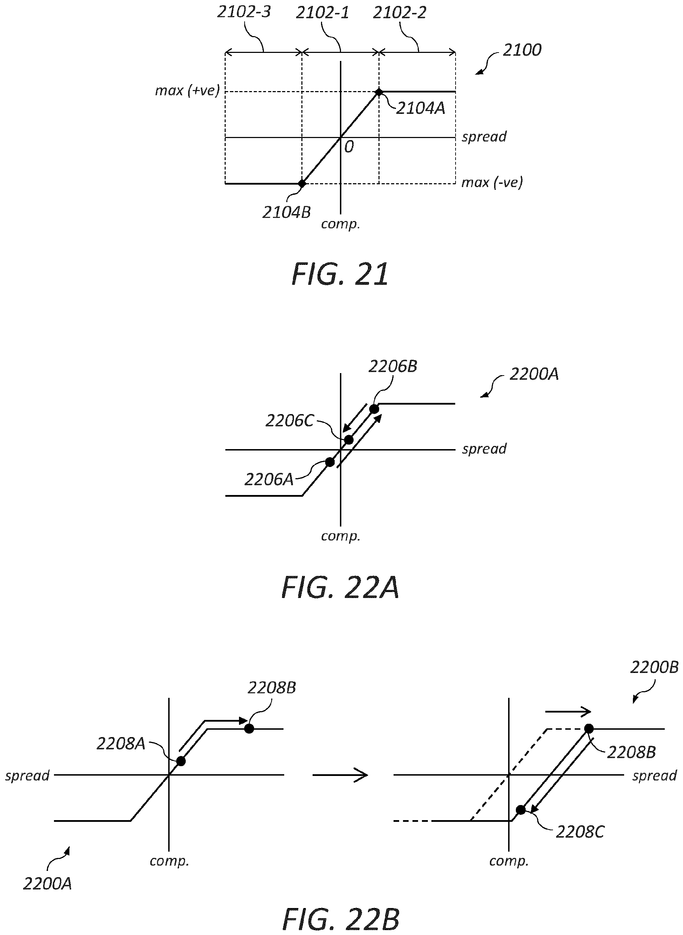

Figure 21 illustrates a first example lost motion compensation function. In Figure 21, the lost motion compensation function is a piecewise function 2100. In Figure 21, the lost motion compensation function is a piecewise linear function - although this need not be the case. In Figure 21, the x-axis is representative of desired or offset-corrected spread angle, and the *y*-axis is representative of lost motion compensation value (e.g. lost motion spread compensation value or lost motion interface position compensation value).

In Figure 21, the first portion of the lost motion compensation function is a first sub-function 2102-1 of the piecewise function 2100 that relates desired or offset-corrected spread angle to lost motion compensation value for a range of desired or offset-corrected spread angles. The first sub-function 2102-1 intersects the x-axis at a lost motion compensation value of zero. Initially (e.g. when a robotic surgical instrument is first installed and driven to an offset-corrected zero configuration), the first sub-function 2102-1 can be set to intersect the *x*-axis at a desired or offset-corrected spread angle of zero - as shown in Figure 21. That said, as explained below, during subsequent use of the robotic surgical instrument the piecewise function 2100 may be translated along the *x*-axis (e.g. horizontally translated) such that the desired or offset-corrected spread angle at which the piecewise function 2100 intersects the *y*-axis changes. In Figure 21, the first sub-function 2102-1 is a linear sub-function. It is to be understood that the first sub-function could have a different gradient to that shown in Figure 21. In other examples, the first sub-function 2102-1 need not be a linear sub-function. For example, the first sub-function could alternatively be a non-linear sub-function. In another example, the first sub-function could itself be a piecewise function comprising two or more linear and/or non-linear sub-sub-functions. A suitable first sub-function 2102-1 can be determined empirically.

In Figure 21, the range of desired or offset-corrected spread angles is between the first endpoint 2104A of the first sub-function 2102-1 and the second endpoint 2104B of the first sub-function 2102-1. The first endpoint 2104A of the first sub-function 2102-1 is at the greatest desired or offset-corrected spread angle within the range of desired or offset-corrected spread angles. The second endpoint 2104B of the first sub-function 2102-1 is at the smallest desired or offset-corrected spread angle within the range of desired or offset-corrected spread angles. A suitable range of desired or offset-corrected spread angles can be determined empirically. In some illustrative examples, it has been empirically determined that, initially (e.g. when a robotic surgical instrument is first installed and driven to an offset-corrected zero configuration), the first sub-function 2102-1 can be set to span a range of desired or offset-corrected spread angles of: -0.5° to +0.5° for a robotic surgical ultrasonic instrument; -1.4° to +1.4° for a robotic surgical needle holder instrument; -1.6° to +1.6° for a robotic surgical fenestrate grasper instrument; and -2.3° to +2.3° for a robotic surgical curved scissor instrument. That said, as explained below, during subsequent use of the robotic surgical instrument, the piecewise function 2100 may be translated along the x-axis (e.g. horizontally translated) such that the desired or offset-corrected spread angles at which the endpoints of the range reside are translated along the x-axis. It is to be understood that the different types of robotic surgical instrument referred to in this paragraph are given by way of example only, and do not represent an exhaustive list of different types of robotic surgical instrument to which the principles described herein could be applied.

In Figure 21, the second portion of the lost motion compensation function is a second sub-function 2102-2 of the piecewise function 2100 that is representative of the maximum positive lost motion compensation value. In Figure 21, the second sub-function 2102-2 is a linear sub-function. In Figure 21, the second sub-function 2102-2 has a gradient of zero. That is, the second sub-function 2102-2 is equal to the maximum positive lost motion compensation value for all values of desired or offset-corrected spread angle. A suitable maximum positive lost motion compensation value can be determined empirically. In some examples, it has been empirically determined that a suitable maximum positive lost motion spread compensation value for: a robotic surgical ultrasonic instrument is +2.5°; a robotic surgical needle holder instrument is +7°; a robotic surgical fenestrate grasper instrument is +8°; and a robotic surgical curved scissor instrument is +11.5°. It is to be understood that the different types of robotic surgical instrument referred to in this paragraph are given by way of example only, and do not represent an exhaustive list of different types of robotic surgical instrument to which the principles described herein could be applied.

In Figure 21, the first transition point of the lost motion compensation function is the first endpoint 2104A of the first sub-function 2102-1 that connects the first sub-function 2102-1 and the second sub-function 2102-2.

In Figure 21, the third portion of the lost motion compensation function is a third sub-function 2102-3 of the piecewise function 2100 that is representative of the maximum negative lost motion compensation value. In Figure 21, the third sub-function 2102-3 is a linear sub-function. In Figure 21, the third sub-function 2102-3 has a gradient of zero. That is, the third sub-function 2102-3 is equal to the maximum negative lost motion compensation value for all values of desired or offset-corrected spread angle. A suitable maximum negative lost motion compensation value can be determined empirically. In some examples, it has been empirically determined that a suitable maximum negative lost motion spread compensation value for: a robotic surgical ultrasonic instrument is -2.5°; a robotic surgical needle holder instrument is -7°; a robotic surgical fenestrate grasper instrument is -8°; and a robotic surgical curved scissor instrument is -11.5°. It is to be understood that the different types of robotic surgical instrument referred to in this paragraph are given by way of example only, and do not represent an exhaustive list of different types of robotic surgical instrument to which the principles described herein could be applied.

In Figure 21, the second transition point of the lost motion compensation function is the second endpoint 2104B of the first sub-function 2102-1 that connects the first sub-function 2102-1 and the third sub-function 2102-3.

In a specific example, the piecewise function 2100 shown in Figure 21 may be represented by Equation 18, as follows:

$$\theta_{comp.} = \theta_{comp.}^{max(+ve)} \ for \ \theta_{desired} \geq \theta_{desired}^{end\ 1}$$

$$\theta_{comp.} = \sigma\left(\theta_{desired} - \theta_{zero\ comp.}\right) for \ \theta_{desired}^{end\ 1} \geq \theta_{desired} \geq \theta_{desired}^{end\ 2}$$

$$\theta_{comp.} = \theta_{comp.}^{max(-ve)} \ for \ \theta_{desired} \leq \theta_{desired}^{end\ 2}$$

$$(18)$$

where $\theta_{comp.}$ is a lost motion spread compensation value, $\theta_{desired}$ is the desired spread angle, $\theta_{zero\ comp.}$ is a desired spread angle for which the lost motion spread compensation value is to equal zero, $\sigma$ is a lost motion sensitivity factor, $\theta_{desired}^{end\ 1}$ is the first endpoint, $\theta_{desired}^{end\ 2}$ is the second endpoint, $\theta_{comp.}^{max(+ve)}$ is the maximum positive lost motion compensation value and $\theta_{comp.}^{max(-ve)}$ is the maximum negative lost motion compensation value.

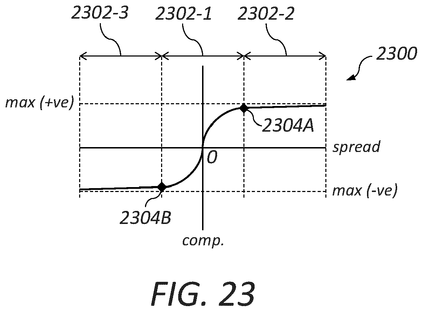

Figure 23 illustrates a second example lost motion compensation function. In Figure 23, the lost motion compensation function is a sigmoid function 2300. A suitable sigmoid function 2300 can be determined empirically. In Figure 23, the x-axis is representative of desired or offset-corrected spread angle, and the y-axis is representative of lost motion compensation value (e.g. lost motion spread compensation value or lost motion interface position compensation value).

In Figure 23, the first portion of the lost motion compensation function is a first section 2302-1 of the sigmoid function 2300 that relates desired or offset-corrected spread angle to lost motion compensation value for a range of desired or offset-corrected spread angles. The first section 2302-1 of the sigmoid function 2300 intersects the x-axis at a lost motion compensation value of zero. Initially (e.g. when a robotic surgical instrument is first installed and driven to an offset-corrected zero configuration), the first section 2302-1 of the sigmoid function 2300 can be set to intersect the x-axis at a desired or offset-corrected spread angle of zero - as shown in Figure 23. That said, as explained below, during subsequent use of the robotic surgical instrument the sigmoid function 2300 may be translated along the x-axis (e.g. horizontally translated) such that the desired or offset-corrected spread angle at which the sigmoid function 2300 intersects the x-axis changes.

In Figure 23, the range of desired or offset-corrected spread angles is between a first point 2304A on the sigmoid function 2300 and a second point 2304B on the sigmoid function 2300. The first point 2304A on the sigmoid function 2300 is at the greatest desired or offset-corrected spread angle within the range of desired or offset-corrected spread angles. The second point 2304B on the sigmoid function 2300 is at the smallest desired or offset-corrected spread angle within the range of desired or offset-corrected spread angles. A suitable range of desired or offset-corrected spread angles can be determined empirically.

In Figure 23, the second portion of the lost motion compensation function is a second section 2302-2 of the sigmoid function 2300 that tends towards the maximum positive lost motion compensation value. A suitable maximum positive lost motion compensation value can be determined empirically. In Figure 23, the first transition point of the lost motion compensation function is the first point 2304A on the sigmoid function 2300. The first point 2304A denotes a boundary (e.g. a hypothetical or theoretical boundary) between the first and second sections 2302-1 and 2302-2 of the sigmoid function 2300. The first point 2304A may be at a predetermined lost motion compensation value. The first point 2304A may be set at a lost motion compensation value that is equal to a predetermined percentage of the maximum positive lost motion compensation value. In examples, the predetermined percentage may be 90%, preferably 95%, or more preferably 98%.

In Figure 23, the third portion of the lost motion compensation function is a third section 2302-3 of the sigmoid function 2300 that tends towards the maximum negative lost motion compensation value. A suitable maximum negative lost motion compensation value can be determined empirically. In Figure 23, the second transition point of the lost motion compensation function is the second point 2304B on the sigmoid function 2300. The second point 2304B denotes a boundary (e.g. a hypothetical or theoretical boundary) between the first and third sections 2302-1 and 2302-3 of the sigmoid function 2300. The second point 2304B may be at a predetermined lost motion compensation value. The second point 2304B may be set at a lost motion compensation value that is equal to a predetermined percentage of the maximum negative lost motion compensation value. In examples, the predetermined percentage may be 90%, preferably 95%, or more preferably 98%.

Different lost motion compensation functions may be suitable for different robotic surgical instruments. That is, lost motion compensation functions of different types (e.g. piecewise, sigmoid or otherwise) may be suitable for different robotic surgical instruments, and/or lost motion compensation functions of the same type but with different parameters (e.g. types of first sub-function, sensitivity factors, first and second transition point values, maximum positive and negative lost motion compensation values, etc.) may be suitable for different robotic surgical instruments. A suitable lost motion compensation function may be determined for each robotic surgical instrument empirically. The control system may store a suitable lost motion compensation function for each robotic surgical instrument and/or each robotic surgical instrument may store its suitable lost motion compensation function in a memory local to that instrument such that that function can be provided to the control system 124 when that instrument is attached to the surgical robot arm.

Figures 22A and 22B illustrate example uses of the first example lost motion compensation function (e.g. as illustrated in Figure 21) to approximate lost motion compensation values.

Figure 22A illustrates how the control system can be configured to use the lost motion compensation function illustrated in Figure 21 when a series of requests are received to drive the spread angle to a series of different desired spread angles within the range of desired or offset-corrected spread angles.

Referring to Figure 22A, the control system can be configured to receive a request to increase the spread angle from a desired or offset-corrected spread angle 2206A within the range of desired or offset-corrected spread angles to an increased desired or offset-corrected spread angle 2206B within the range of desired or offset-corrected spread angles. In response, the control system can be configured to approximate a lost motion compensation value for the increased desired or offset-corrected spread angle 2206B using the first portion of the lost motion compensation function 2200A. The control system can be configured to subsequently receive a request to decrease the spread angle from the increased desired or offset-corrected spread angle 2206B to a decreased desired or offset-corrected spread angle 2206C within the range of desired or offset-corrected spread angles. In other words, the control system can be configured to subsequently receive a request to change the direction in which the first and second end effector elements are being driven (e.g. from opening to closing). In response, the control system can be configured to approximate a lost motion compensation value for the decreased desired or offset-corrected spread angle 2206C using the first portion of the lost motion compensation function 2200A. That is, when both the increased desired or offset-corrected spread angle 2206B and the subsequently decreased desired or offset-corrected spread angle 2206C are within the range of desired or offset-corrected spread angles, the lost motion compensation value for the decreased desired or offset-corrected spread angle 2206C can be approximated using the same lost motion compensation function 2200A as is used to approximate the lost motion compensation value for the increased desired or offset-corrected spread angle 2206B.

Likewise (not shown in Figure 22A), the control system can be configured to receive a request to decrease the spread angle from a desired or offset-corrected spread angle within the range of desired or offset-corrected spread angles to a decreased desired or offset-corrected spread angle within the range of desired or offset-corrected spread angles. In response, the control system can be configured to approximate a lost motion compensation value for the decreased desired or offset-corrected spread angle using the first portion of the lost motion compensation function. The control system can be configured to subsequently receive a request to increase the spread angle from the decreased desired or offset-corrected spread angle to an increased desired or offset-corrected spread angle within the range of desired or offset-corrected spread angles. In other words, the control system can be configured to subsequently receive a request to change the direction in which the first and second end effector elements are being driven (e.g. from closing to opening). In response, the control system can be configured to approximate a lost motion compensation value for the increased desired or offset-corrected spread angle using the first portion of the lost motion compensation function. That is, when both the decreased desired or offset-corrected spread angle and the subsequently increased desired or offset-corrected spread angle are within the range of desired or offset-corrected spread angles, the lost motion compensation value for the increased desired or offset-corrected spread angle can be approximated using the same lost motion compensation function as is used to approximate the lost motion compensation value for the decreased desired or offset-corrected spread angle.

Figure 22B illustrates how the control system can be configured to use the lost motion compensation function illustrated in Figure 21 when a series of requests are received to drive the spread angle to a series of different desired spread angles, and at least one of those desired spread angles is outside of the range of desired or offset-corrected spread angles.

Referring to Figure 22B, the control system can be configured to receive a request to increase the spread angle from a desired or offset-corrected spread angle 2208A within the range of desired or offset-corrected spread angles to an increased desired or offset-corrected spread angle 2208B that is greater than the greatest desired or offset-corrected spread angle within the range of desired or offset-corrected spread angles. In response, the control system can be configured to determine a lost motion compensation value for the increased desired or offset-corrected spread angle 2208B using the second portion of the lost motion compensation function 2200A that is representative of a maximum positive lost motion compensation value. In examples where the control system uses the lost motion compensation function illustrated in Figure 21, the control system would determine the maximum positive lost motion compensation value as the lost motion compensation value for the increased desired or offset-corrected spread angle 2208B. The control system can be configured to subsequently receive a request to decrease the spread angle from the increased desired or offset-corrected spread angle 2208B to a decreased desired or offset-corrected spread angle 2208C that is less than the increased desired or offset-corrected spread angle 2208B. In other words, the control system can be configured to subsequently receive a request to change the direction in which the first and second end effector elements are being driven (e.g. from opening to closing). The decreased desired or offset-corrected spread angle 2208C may be any amount less than the increased desired or offset-corrected spread angle 2208B. For example, the decreased desired or offset-corrected spread angle 2208C may be: greater than the greatest desired or offset-corrected spread angle within the range of desired or offset-corrected spread angles (not shown in Figure 22B); within the range of desired or offset-corrected spread angles (as shown in Figure 22B); or less than the smallest desired or offset-corrected spread angle within the range of desired or offset-corrected spread angles (not shown in Figure 22B). In response, the control system can be configured to modify the lost motion compensation function 2200A by translating the lost motion compensation function such that the first transition point is shifted to the increased desired or offset-corrected spread angle 2208B. For example, as shown on the right-handside of Figure 22B, this can be achieved by translating the lost motion compensation function along the x-axis - e.g. "to the right". In examples where the control system uses the lost motion compensation function represented by Equation 18, this could be achieved by increasing the x-values of $\theta_{zero\ comp.}$, $\theta_{desired}^{end\ 1}$ and $\theta_{desired}^{end\ 2}$. For example, each of those values could be increased by the desired or offset-corrected spread angle difference between greatest desired or offset-corrected spread angle within the range of desired or offset-corrected spread angles (e.g. the x-value of $\theta_{desired}^{end\ 1}$ of the lost motion compensation function 2200A) and the increased desired or offset-corrected spread angle (e.g. the x-value of 2208B). Modifying the lost motion compensation function may not comprise translating the lost motion compensation function along the $y$-axis - e.g. "up" or "down". The output of this modifying step is a modified lost motion compensation function 2200B. The control system can be configured to approximate a lost motion compensation value for the decreased desired or offset-corrected spread angle 2208C using the modified lost motion compensation function 2200B. In the example shown in Figure 22B, the control system can be configured to approximate a lost motion compensation value for the decreased desired or offset-corrected spread angle 2208C using the modified first portion of the modified lost motion compensation function, as the decreased desired or offset-corrected spread angle 2208C is within the modified range of desired or offset-corrected spread angles - although this need not always be the case. Note that, in Figure 22B, the decreased desired or offset-corrected spread angle 2208C has the same desired or offset-corrected spread angle value as the desired or offset-corrected spread angle 2208A - however the lost motion compensation value approximated for the decreased desired or offset-corrected spread angle 2208C using the modified lost motion compensation function 2200B will be different to the lost motion compensation value approximated for the desired or offset-corrected spread angle 2208A using the lost motion compensation function 2200A.

Likewise (not shown in Figure 22B), the control system can be configured to receive a request to decrease the spread angle from a desired or offset-corrected spread angle within the range of desired or offset-corrected spread angles to a decreased desired or offset-corrected spread angle that is less than the smallest desired or offset-corrected spread angle within the range of desired or offset-corrected spread angles. In response, the control system can be configured to determine a lost motion compensation value for the decreased desired or offset-corrected spread angle using the third portion of the lost motion compensation function that is representative of a maximum negative lost motion compensation value. In examples where the control system uses the lost motion compensation function illustrated in Figure 21, the control system would determine the maximum negative lost motion compensation value as the lost motion compensation value for the decreased desired or offset-corrected spread angle. The control system can be configured to subsequently receive a request to increase the spread angle from the decreased desired or offset-corrected spread angle to an increased desired or offset-corrected spread angle that is greater than the decreased desired or offset-corrected spread angle. In other words, the control system can be configured to subsequently receive a request to change the direction in which the first and second end effector elements are being driven (e.g. from closing to opening). The increased desired or offset-corrected spread angle may be any amount more than the decreased desired or offset-corrected spread angle. For example, the increased desired or offset-corrected spread angle may be: less than the smallest desired or offset-corrected spread angle within the range of desired or offset-corrected spread angles; within the range of desired or offset-corrected spread angles; or greater than the greatest desired or offset-corrected spread angle within the range of desired or offset-corrected spread angles. In response, the control system can be configured to modify the lost motion compensation function by translating the lost motion compensation function such that the second transition point is shifted to the decreased desired or offset-corrected spread angle. For example, this can be achieved by translating the lost motion compensation function along the x-axis - e.g. "to the left". In examples where the control system uses the lost motion compensation function represented by Equation 18, this could be achieved by decreasing the x-values of $\theta_{zero\ comp.}$, $\theta_{desired}^{end\ 1}$ and $\theta_{desired}^{end\ 2}$. For example, each of those values could be decreased by the desired or offset-corrected spread angle difference between smallest desired or offset-corrected spread angle within the range of desired or offset-corrected spread angles and the decreased desired or offset-corrected spread angle. Modifying the lost motion compensation function may not comprise translating the lost motion compensation function along the *y*-axis - e.g. "up" or "down". The output of this modifying step is a modified lost motion compensation function. The control system can be configured to approximate a lost motion compensation value for the increased desired or offset-corrected spread angle using the modified lost motion compensation function.

For completeness, not shown in the Figures, the control system can be configured to receive a request to increase the spread angle from a desired or offset-corrected spread angle within the range of desired or offset-corrected spread angles to an increased desired or offset-corrected spread angle that is greater than the greatest desired or offset-corrected spread angle within the range of desired or offset-corrected spread angles. As above, in response, the control system can be configured to determine a lost motion compensation value for the increased desired or offset-corrected spread angle using the second portion of the lost motion compensation function that is representative of a maximum positive lost motion compensation value. The control system can be configured to subsequently receive a request to further increase the spread angle from the increased desired or offset-corrected spread angle to a further increased desired or offset-corrected spread angle. In response, the control system can be configured to determine a lost motion compensation value for the further increased desired or offset-corrected spread angle using the second portion of the lost motion compensation function (e.g. without modifying the lost motion compensation function).

Likewise, not shown in the Figures, the control system can be configured to receive a request to decrease the spread angle from a desired or offset-corrected spread angle within the range of desired or offset-corrected spread angles to a decreased desired or offset-corrected spread angle that is less than the smallest desired or offset-corrected spread angle within the range of desired or offset-corrected spread angles. As above, in response, the control system can be configured to determine a lost motion compensation value for the decreased desired or offset-corrected spread angle using the third portion of the lost motion compensation function that is representative of a maximum negative lost motion compensation value. The control system can be configured to subsequently receive a request to further decrease the spread angle from the decreased desired or offset-corrected spread angle to a further decreased desired or offset-corrected spread angle. In response, the control system can be configured to determine a lost motion compensation value for the decreased desired or offset-corrected spread angle using the third portion of the lost motion compensation function (e.g. without modifying the lost motion compensation function).

Although an illustrated example is not shown in the Figures or separately described for conciseness, it will be appreciated that the lost motion compensation function illustrated in Figure 23 can be used by the control system in an analogous manner to the lost motion compensation function illustrated in Figure 21 - e.g. as described herein with reference to Figures 22A and 22B.

Returning to Figure 20, in step 2006, the control system is configured to control the surgical robot arm in dependence on the desired spread angle and the lost motion compensation value. That is, the control system is configured to control the surgical robot arm to control the spread angle between the first end effector element and the second end effector element of the robotic surgical instrument in dependence on the desired spread angle and the lost motion compensation value.

The control system can be configured to determine a commanded spread angle in dependence on the desired spread angle, an offset value determined using any of the sets of steps described herein with reference to Figures 9, 10, 13, 14 and 19 and the lost motion compensation value. The control system can be configured to control the surgical robot arm to drive the robotic surgical instrument using the commanded spread angle.

In a first example, determining the commanded spread angle in dependence on the desired spread angle, the offset value and the lost motion compensation value may comprise: determining an offset-corrected spread angle in dependence on the desired spread angle and the offset value (e.g. by summing the desired spread angle and the offset value), approximating a lost motion compensation value in dependence on the offset-corrected spread angle and the lost motion compensation function, and determining the commanded spread angle in dependence on the offset-corrected spread angle and the lost motion compensation value. For example, the commanded spread angle may be determined by summing the offset-corrected spread angle and the lost motion compensation value.

In a second example, determining the commanded spread angle in dependence on the desired spread angle, the offset value and the lost motion compensation value may comprise: approximating a lost motion compensation value in dependence on the desired spread angle and the lost motion compensation function, and determining the commanded spread angle in dependence on the desired spread angle, the offset value and the lost motion compensation value. For example, the commanded spread angle may be determined by summing the desired spread angle, the offset value and the lost motion compensation value.

The amount of lost motion is typically greatest at the instant in time when the direction in which the first and second end effector elements are being driven is changed (e.g. from opening to closing, or vice versa). Hence, it is desirable for the lost motion compensation value to change when the direction in which the first and second end effector elements are being driven is changed. That said, significant instantaneous changes to the lost motion compensation value can be perceived by a surgeon as causing jerky or sudden movements of the end effector elements - which is undesirable, and sometimes unsettling for a surgeon. The inventors have found that configuring the control system to perform steps 2004 and 2006 in the manner described herein offers a good compromise between changing the lost motion compensation value enough to address the lost motion occurring when the direction in which the first and second end effector elements are being driven is changed, yet not changing the lost motion compensation value so much as to cause movements of the end effector elements that a surgeon might perceive as jerky or sudden.

It is to be understood that the set of steps described herein with reference to Figure 20 could be performed independently of (i.e. need not necessarily be performed in combination with) the sets of steps described herein with reference to Figures 9, 10, 13, 14 and 19. For example, the control system can be configured to receive a request to drive the spread angle between the first end effector element and the second end effector element to a desired spread angle. The control system can be configured to determine a commanded spread angle in dependence on the desired spread angle and the lost motion compensation value. Determining the commanded spread angle in dependence on the desired spread angle and the lost motion compensation value may comprise: approximating a lost motion compensation value in dependence on the desired spread angle and the lost motion compensation function (e.g. where the lost motion compensation function relates desired spread angle to lost motion compensation value), and determining the commanded spread angle in dependence on the desired spread angle and the lost motion compensation value. For example, the commanded spread angle may be determined by summing the desired spread angle and the lost motion compensation value. The control system can be configured to control the surgical robot arm to drive the robotic surgical instrument using the commanded spread angle.

It is to be understood that the principles described herein can be applied to control systems for controlling robot arms used for purposes other than surgery. For example, the robot arm may be used on a vehicle assembly line.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features. In view of the foregoing description it will be evident to a person skilled in the art that various modifications may be made within the scope of the invention.

ANNEX

Aspects of the disclosure are described in the numbered annex paragraphs of the Annex in terms of exemplary combinations of features.

1. A control system for controlling a surgical robot arm, the surgical robot arm having a drive mechanism configured for driving a robotic surgical instrument, said robotic surgical instrument comprising a first end effector element and a second end effector element, the drive mechanism comprising one or more interface elements configured to interface with the robotic surgical instrument for controlling a spread angle between the first end effector element and the second end effector element, the control system being configured to:

cause an instrument drive force applied at the one or more interface elements to be varied;
measure, at each of a plurality of times, the instrument drive force applied at the one or more interface elements;
determine, for each of the plurality of times, a force behaviour value using a derivative of the measured instrument drive force with respect to the time at that time;
determine an offset value in dependence on the plurality of determined force behaviour values; and
control, in dependence on the offset value, the surgical robot arm to control the spread angle between the first end effector element and the second end effector element of the robotic surgical instrument.

2. The control system of annex paragraph 1, the control system being configured to use the offset value to determine a calibrated zero configuration for the robotic surgical instrument, the calibrated zero configuration being:

when the control system is configured to cause the instrument drive force to be varied to close the robotic surgical instrument from an open configuration, the configuration in which an inner surface of the first end effector element first contacts an inner surface of the second end effector element; or
when the control system is configured to cause the instrument drive force to be varied to open the robotic surgical instrument from a closed configuration, the configuration in which an inner surface of the first end effector element last contacts an inner surface of the second end effector element.

3. The control system of annex paragraph 1 or 2, the control system being configured to:

cause the instrument drive force to be varied to close the robotic surgical instrument from an open configuration; and/or
cause the instrument drive force to be varied to open the robotic surgical instrument from a closed configuration.

4. The control system of any of annex paragraphs 1 to 3, the control system being configured to:

determine a further offset value in dependence on the plurality of determined force behaviour values;
combine the offset value and the further offset value to form a combined offset value; and
control, in dependence on the combined offset value, the surgical robot arm to control the spread angle between the first end effector element and the second end effector element of the robotic surgical instrument.

5. The control system of any preceding annex paragraph, the control system being configured to cause the instrument drive force applied at the one or more interface elements to be varied such that each of the one or more interface elements move at a substantially constant velocity.

6. The control system of any preceding annex paragraph, wherein the offset value is a spread offset value, the control system being further configured to:

measure, at each of the plurality of times, a position of each of the one or more interface elements;
predict, for each of the plurality of times, a resultant spread angle between the first end effector element and the second end effector element in dependence on the measured position of each of the one or more interface elements at that time; and
determine the spread offset value in dependence on the plurality of determined force behaviour values and the

*plurality of predicted resultant spread angles.*

7. The control system of annex paragraph 6, the control system being configured to:

cause the instrument drive force to be varied to close the robotic surgical instrument from an open configuration, and approximate in dependence on the plurality of determined force behaviour values a time at which the force behaviour value for that time reaches or exceeds a force behaviour threshold value; or
cause the instrument drive force to be varied to open the robotic surgical instrument from a closed configuration, and approximate in dependence on the plurality of determined force behaviour values a time at which the force behaviour value for that time reaches or falls below a force behaviour threshold value; and
determine, as the spread offset value, the resultant spread angle predicted for that time in dependence on the plurality of predicted resultant spread angles.

8. The control system of annex paragraphs 6 or 7, the control system being configured to use the spread offset value to adjust subsequently predicted resultant spread angles.

9. The control system of any of annex paragraphs 6 to 8, the control system being further configured to:

determine, for each of the plurality of predicted resultant spread angles, an instrument stiffness value using a derivative of the measured instrument drive force with respect to the predicted resultant spread angle at that predicted resultant spread angle;
determine a further spread offset value in dependence on the plurality of determined instrument stiffness values;
combine the spread offset value and the further spread offset value to form a combined spread offset value; and
control, in dependence on the combined spread offset value, the surgical robot arm to control the spread angle between the first end effector element and the second end effector element of the robotic surgical instrument.

10. The control system of any of annex paragraphs 6 to 8, the control system being further configured to:

fit a linear relationship to at least two measured instrument drive force and predicted resultant spread angle data pairs, wherein a data pair comprises an instrument drive force measured at a time of the plurality of times and a resultant spread angle predicted for the same time of the plurality of times;
determine a further spread offset value, wherein determining the further spread offset value comprises approximating, using the linear relationship, a predicted spread angle when the measured instrument drive force would equal a predetermined force value;
combine the spread offset value and the further spread offset value to form a combined spread offset value; and
control, in dependence on the combined spread offset value, the surgical robot arm to control the spread angle between the first end effector element and the second end effector element of the robotic surgical instrument.

11. The control system of any of annex paragraphs 1 to 5, wherein the offset value is an interface position offset value, the control system being further configured to:

measure, at each of the plurality of times, a position of an interface element of the one or more interface elements that is indicative of a predicted spread angle between the first end effector element and the second end effector element; and
determine the interface position offset value in dependence on the plurality of determined force behaviour values and the plurality of measured interface positions.

12. The control system of annex paragraph 11, the control system being configured to:

cause the instrument drive force to be varied to close the robotic surgical instrument from an open configuration, and approximate in dependence on the plurality of determined force behaviour values a time at which the force behaviour value for that time reaches or exceeds a force behaviour threshold value; or
cause the instrument drive force to be varied to open the robotic surgical instrument from a closed configuration, and approximate in dependence on the plurality of determined force behaviour values a time at which the force behaviour value for that time reaches or falls below a force behaviour threshold value; and
determine, as the interface position offset value, the interface position at that time in dependence on the plurality of measured interface positions.

13. The control system of annex paragraph 11 or 12, the control system being configured to use the interface position offset value to adjust subsequently measured positions of the interface element.

14. The control system of any of annex paragraphs 11 to 13, the control system being further configured to:

determine, for each of the plurality of measured interface positions, an instrument stiffness value using a derivative of the measured instrument drive force with respect to the measured interface position at that measured interface position;
determine a further interface position offset value in dependence on the plurality of determined instrument stiffness values;
combine the interface position offset value and the further interface position offset value to form a combined interface position offset value; and
control, in dependence on the combined interface position offset value, the surgical robot arm to control the spread angle between the first end effector element and the second end effector element of the robotic surgical instrument.

15. The control system of any of annex paragraphs 11 to 13, the control system being further configured to:

fit a linear relationship to at least two measured instrument drive force and measured interface position data pairs, wherein a data pair comprises an instrument drive force measured at a time of the plurality of times and an interface position measured at the same time of the plurality of times;
determine a further interface position offset value, wherein determining the further interface position offset value comprises approximating, using the linear relationship, an interface position when the measured instrument drive force would equal a predetermined force value;
combine the interface position offset value and the further interface position offset value to form a combined interface position offset value; and
control, in dependence on the combined interface position offset value, the surgical robot arm to control the spread angle between the first end effector element and the second end effector element of the robotic surgical instrument.

16. The control system of any preceding annex paragraph, wherein the derivative of the measured instrument drive force with respect to the time at that time is the first derivative of the measured instrument drive force with respect to the time at that time.

17. The control system of annex paragraph 16, wherein the first derivative of the measured instrument drive force with respect to time at a time, $t_n$, is determined by:

assessing the difference between the measured instrument drive force, $F_n$, at that time, $t_n$, and the measured instrument drive force $F_{n-1}$, at a preceding time, $t_{n-1}$, and dividing that difference, $(F_n - F_{n-1})$, by the time difference between the time $t_n$, and the time $t_{n-1}$; or
assessing the difference between the measured instrument drive force, $F_{n+1}$, at a subsequent time, $t_{n+1}$, and the measured instrument drive force $F_n$, at that time, $t_n$, and dividing that difference, $(F_{n+1} - F_n)$, by the time difference between the time $t_{n+1}$, and the time $t_n$; or
assessing the difference between the measured instrument drive force, $F_{n+1}$, at a subsequent time, $t_{n+1}$, and the measured instrument drive force $F_{n-1}$, at a preceding time, $t_{n-1}$, and dividing that difference, $(F_{n+1} - F_{n-1})$, by the time difference between the time $t_{n+1}$, and the time $t_{n-1}$.

18. The control system of any preceding annex paragraph, wherein:

the measured instrument drive force is dependent on a first force measured at a first interface element for changing an angle of the first end effector element and a second force measured at a second interface element for changing an angle of the second end effector element, optionally wherein the instrument drive force is dependent on a sum of the first force and the second force; or
the measured instrument drive force is dependent on a force measured at a single interface element for changing an angle of one or both of the first end effector element and the second end effector element.

19. The control system of any preceding annex paragraph, wherein the plurality of measured instrument drive forces are filtered prior to being used to determine the plurality of force behaviour values.

20. The control system of any preceding annex paragraph, the control system being further configured to:

receive a request to drive the spread angle between the first end effector element and the second end effector element to a desired spread angle;
approximate a lost motion compensation value in dependence on a lost motion compensation function; and
control, in dependence on the desired spread angle and the lost motion compensation value, the surgical robot arm to control the spread angle between the first end effector element and the second end effector element of the robotic surgical instrument.

21. The control system of annex paragraph 20, wherein the lost motion compensation function comprises:

a first portion that relates desired or offset-corrected spread angle to lost motion compensation value for a range of desired or offset-corrected spread angles; and
a second portion that is representative of a maximum positive lost motion compensation value; wherein the first portion connects to the second portion at a first transition point, the first transition point being at the greatest desired or offset-corrected spread angle within the range of desired or offset-corrected spread angles.

22. The control system of annex paragraph 21, the control system being configured to:

receive a request to increase the spread angle from a desired or offset-corrected spread angle within the range of desired or offset-corrected spread angles to an increased desired or offset-corrected spread angle within the range of desired or offset-corrected spread angles;
approximate a lost motion compensation value for the increased desired or offset-corrected spread angle using the first portion of the lost motion compensation function;
subsequently, receive a request to decrease the spread angle from the increased desired or offset-corrected spread angle to a decreased desired or offset-corrected spread angle within the range of desired or offset-corrected spread angles; and
approximate a lost motion compensation value for the decreased desired or offset-corrected spread angle using the first portion of the lost motion compensation function.

23. The control system of annex paragraph 21 or 22, the control system being configured to:

receive a request to increase the spread angle from a desired or offset-corrected spread angle within the range of desired or offset-corrected spread angles to an increased desired or offset-corrected spread angle that is greater than the greatest desired or offset-corrected spread angle within the range of desired or offset-corrected spread angles;
determine a lost motion compensation value for the increased desired or offset-corrected spread angle using the second portion of the lost motion compensation function that is representative of a maximum positive lost motion compensation value;
subsequently, receive a request to decrease the spread angle from the increased desired or offset-corrected spread angle to a decreased desired or offset-corrected spread angle that is less than the increased desired or offset-corrected spread angle;
modify the lost motion compensation function by translating the lost motion compensation function such that the first transition point is shifted to the increased desired or offset-corrected spread angle; and
approximate a lost motion compensation value for the decreased desired or offset-corrected spread angle using the modified lost motion compensation function.

24. The control system of any of annex paragraphs 21 to 23, wherein the lost motion compensation function is a piecewise function, in which:

the first portion of the lost motion compensation function is a first sub-function of the piecewise function that relates desired or offset-corrected spread angle to lost motion compensation value for the range of desired or offset-corrected spread angles;
the second portion of the lost motion compensation function is a second sub-function of the piecewise function that is representative of the maximum positive lost motion compensation value; and
the first transition point of the lost motion compensation function is a first endpoint of the first sub-function that connects the first sub-function and the second sub-function.

25. The control system of any of annex paragraphs 21 to 23, wherein the lost motion compensation function is a sigmoid function, in which:

the first portion of the lost motion compensation function is a first section of the sigmoid function that relates desired or offset-corrected spread angle to lost motion compensation value for a range of desired or offset-corrected spread angles;

the second portion of the lost motion compensation function is a second section of the sigmoid function that tends towards the maximum positive lost motion compensation value; and

the first transition point of the lost motion compensation function is a first point on the sigmoid function that denotes a boundary between the first and second sections of sigmoid function, the first point being at a predetermined lost motion compensation value.

26. The control system of any of annex paragraphs 20 to 25, wherein the lost motion compensation function comprises:

a first portion that relates desired or offset-corrected spread angle to lost motion compensation value for a range of desired or offset-corrected spread angles; and

a third portion that is representative of a maximum negative lost motion compensation value; wherein the first portion connects to the third portion at a second transition point, the second transition point being at the smallest desired or offset-corrected spread angle within the range of desired or offset-corrected spread angles.

27. The control system of annex paragraph 26, the control system being configured to:

receive a request to decrease the spread angle from a desired or offset-corrected spread angle within the range of desired or offset-corrected spread angles to a decreased desired or offset-corrected spread angle within the range of desired or offset-corrected spread angles;

approximate a lost motion compensation value for the decreased desired or offset-corrected spread angle using the first portion of the lost motion compensation function;

subsequently, receive a request to increase the spread angle from the decreased desired or offset-corrected spread angle to an increased desired or offset-corrected spread angle within the range of desired or offset-corrected spread angles; and

approximate a lost motion compensation value for the increased desired or offset-corrected spread angle using the first portion of the lost motion compensation function.

28. The control system of annex paragraph 26 or 27, the control system being configured to:

receive a request to decrease the spread angle from a desired or offset-corrected spread angle within the range of desired or offset-corrected spread angles to a decreased desired or offset-corrected spread angle that is less than the smallest desired or offset-corrected spread angle within the range of desired or offset-corrected spread angles;

determine a lost motion compensation value for the decreased desired or offset-corrected spread angle using the third portion of the lost motion compensation function that is representative of a maximum negative lost motion compensation value;

subsequently, receive a request to increase the spread angle from the decreased desired or offset-corrected spread angle to an increased desired or offset-corrected spread angle that is greater than the decreased desired or offset-corrected spread angle;

modify the lost motion compensation function by translating the lost motion compensation function such that the second transition point is shifted to the decreased desired or offset-corrected spread angle; and

approximate a lost motion compensation value for the increased desired or offset-corrected spread angle using the modified lost motion compensation function.

29. The control system of any of annex paragraphs 20 to 28, the control system being further configured to:

determine a commanded spread angle in dependence on the desired spread angle, the offset value and the lost motion compensation value; and

control the surgical robot arm to drive the robotic surgical instrument using the commanded spread angle.

30. The control system of annex paragraph 29, wherein determining the commanded spread angle in dependence on

*the desired spread angle, the offset value and the lost motion compensation value comprises:*

> *determining an offset-corrected spread angle in dependence on the desired spread angle and the offset value, approximating a lost motion compensation value in dependence on the offset-corrected spread angle and the lost motion compensation function, and determining the commanded spread angle in dependence on the offset-corrected spread angle and the lost motion compensation value; or*
> *approximating a lost motion compensation value in dependence on the desired spread angle and the lost motion compensation function, and determining the commanded spread angle in dependence on the desired spread angle, the offset value and the lost motion compensation value.*

*31. The control system of any of annex paragraphs 20 to 30, wherein the lost motion compensation value is a lost motion spread compensation value or a lost motion interface position compensation value.*

*32. A surgical robotic system comprising:*

> *a surgical robot arm having a drive mechanism configured for driving a robotic surgical instrument, said robotic surgical instrument comprising a first end effector element and a second end effector element, the drive mechanism comprising one or more interface elements configured to interface with the robotic surgical instrument for controlling a spread angle between the first end effector element and the second end effector element; and*
> *a control system according to any of annex paragraphs 1 to 31.*

*33. A method of controlling a surgical robot arm, the surgical robot arm having a drive mechanism configured for driving a robotic surgical instrument, said robotic surgical instrument comprising a first end effector element and a second end effector element, the drive mechanism comprising one or more interface elements configured to interface with the robotic surgical instrument for controlling a spread angle between the first end effector element and the second end effector element, the method comprising:*

> *causing an instrument drive force applied at the one or more interface elements to be varied;*
> *measuring, at each of a plurality of times, the instrument drive force applied at the one or more interface elements;*
> *determining, for each of the plurality of times, a force behaviour value using a derivative of the measured instrument drive force with respect to the time at that time;*
> *determining an offset value in dependence on the plurality of determined force behaviour values; and*
> *controlling, in dependence on the offset value, the surgical robot arm to control the spread angle between the first end effector element and the second end effector element of the robotic surgical instrument.*

## Claims

1. A control system for controlling a surgical robot arm, the surgical robot arm having a drive mechanism configured for driving a robotic surgical instrument, said robotic surgical instrument comprising a first end effector element and a second end effector element, the drive mechanism comprising one or more interface elements configured to interface with the robotic surgical instrument for controlling a spread angle between the first end effector element and the second end effector element, the control system being configured to:

   cause an instrument drive force applied at the one or more interface elements to be varied;
   measure, at each of a plurality of times, the instrument drive force applied at the one or more interface elements;
   determine, for each of the plurality of times, a force behaviour value using a derivative of the measured instrument drive force with respect to the time at that time;
   determine an offset value in dependence on the plurality of determined force behaviour values; and
   control, in dependence on the offset value, the surgical robot arm to control the spread angle between the first end effector element and the second end effector element of the robotic surgical instrument.

2. The control system of claim 1, the control system being configured to use the offset value to determine a calibrated zero configuration for the robotic surgical instrument, the calibrated zero configuration being:

   when the control system is configured to cause the instrument drive force to be varied to close the robotic surgical instrument from an open configuration, the configuration in which an inner surface of the first end effector element first contacts an inner surface of the second end effector element; or
when the control system is configured to cause the instrument drive force to be varied to open the robotic surgical instrument from a closed configuration, the configuration in which an inner surface of the first end effector element last contacts an inner surface of the second end effector element.

3. The control system of claim 1 or 2, the control system being configured to:

cause the instrument drive force to be varied to close the robotic surgical instrument from an open configuration; and/or
cause the instrument drive force to be varied to open the robotic surgical instrument from a closed configuration.

4. The control system of any of claims 1 to 3, the control system being configured to:

determine a further offset value in dependence on the plurality of determined force behaviour values;
combine the offset value and the further offset value to form a combined offset value; and
control, in dependence on the combined offset value, the surgical robot arm to control the spread angle between the first end effector element and the second end effector element of the robotic surgical instrument.

5. The control system of any preceding claim, wherein the offset value is a spread offset value, the control system being further configured to:

measure, at each of the plurality of times, a position of each of the one or more interface elements;
predict, for each of the plurality of times, a resultant spread angle between the first end effector element and the second end effector element in dependence on the measured position of each of the one or more interface elements at that time; and
determine the spread offset value in dependence on the plurality of determined force behaviour values and the plurality of predicted resultant spread angles.

6. The control system of claim 5, the control system being configured to:

cause the instrument drive force to be varied to close the robotic surgical instrument from an open configuration, and approximate in dependence on the plurality of determined force behaviour values a time at which the force behaviour value for that time reaches or exceeds a force behaviour threshold value; or
cause the instrument drive force to be varied to open the robotic surgical instrument from a closed configuration, and approximate in dependence on the plurality of determined force behaviour values a time at which the force behaviour value for that time reaches or falls below a force behaviour threshold value; and

determine, as the spread offset value, the resultant spread angle predicted for that time in dependence on the plurality of predicted resultant spread angles.

7. The control system of any of claims 1 to 4, wherein the offset value is an interface position offset value, the control system being further configured to:

measure, at each of the plurality of times, a position of an interface element of the one or more interface elements that is indicative of a predicted spread angle between the first end effector element and the second end effector element; and
determine the interface position offset value in dependence on the plurality of determined force behaviour values and the plurality of measured interface positions.

8. The control system of claim 7, the control system being configured to:

cause the instrument drive force to be varied to close the robotic surgical instrument from an open configuration, and approximate in dependence on the plurality of determined force behaviour values a time at which the force behaviour value for that time reaches or exceeds a force behaviour threshold value; or
cause the instrument drive force to be varied to open the robotic surgical instrument from a closed configuration, and approximate in dependence on the plurality of determined force behaviour values a time at which the force behaviour value for that time reaches or falls below a force behaviour threshold value; and

determine, as the interface position offset value, the interface position at that time in dependence on the plurality of measured interface positions.

9. The control system of any preceding claim, wherein the derivative of the measured instrument drive force with respect to the time at that time is the first derivative of the measured instrument drive force with respect to the time at that time, optionally wherein the first derivative of the measured instrument drive force with respect to time at a time, $t_n$, is determined by:

assessing the difference between the measured instrument drive force, $F_n$, at that time, $t_n$, and the measured instrument drive force $F_{n-1}$, at a preceding *time*, $t_{n-1}$, and dividing that difference, $(F_n - F_{n-1})$, by the time difference between the time $t_n$, and the time $t_{n-1}$; or

assessing the difference between the measured instrument drive force, $F_{n+1}$, at a subsequent time, $t_{n+1}$, and the measured instrument drive force $F_n$, at that time, $t_n$, and dividing that difference, $(F_{n+1} - F_n)$, by the time difference between the time $t_{n+1}$, and the time $t_n$; or

assessing the difference between the measured instrument drive force, $F_{n+1}$, at a subsequent time, $t_{n+1}$, and the measured instrument drive force $F_{n-1}$, at a preceding *time*, $t_{n-1}$, and dividing that difference, $(F_{n+1} - F_{n-1})$, by the time difference between the time $t_{n+1}$, and the time $t_{n-1}$.

10. The control system of any preceding claim, wherein:

the measured instrument drive force is dependent on a first force measured at a first interface element for changing an angle of the first end effector element and a second force measured at a second interface element for changing an angle of the second end effector element, optionally wherein the instrument drive force is dependent on a sum of the first force and the second force; or

the measured instrument drive force is dependent on a force measured at a single interface element for changing an angle of one or both of the first end effector element and the second end effector element.

11. The control system of any preceding claim, the control system being further configured to:

receive a request to drive the spread angle between the first end effector element and the second end effector element to a desired spread angle;

approximate a lost motion compensation value in dependence on a lost motion compensation function; and

control, in dependence on the desired spread angle and the lost motion compensation value, the surgical robot arm to control the spread angle between the first end effector element and the second end effector element of the robotic surgical instrument.

12. The control system of claim 11, wherein:

the lost motion compensation function comprises:

a first portion that relates desired or offset-corrected spread angle to lost motion compensation value for a range of desired or offset-corrected spread angles; and

a second portion that is representative of a maximum positive lost motion compensation value;

wherein the first portion connects to the second portion at a first transition point, the first transition point being at the greatest desired or offset-corrected spread angle within the range

of desired or offset-corrected spread angles; and

the control system is configured to:

receive a request to increase the spread angle from a desired or offset-corrected spread angle within the range of desired or offset-corrected spread angles to an increased desired or offset-corrected spread angle that is greater than the greatest desired or offset-corrected spread angle within the range of desired or offset-corrected spread angles;

determine a lost motion compensation value for the increased desired or offset-corrected spread angle using the second portion of the lost motion compensation function that is representative of a maximum positive lost motion compensation value;

subsequently, receive a request to decrease the spread angle from the increased desired or offset-corrected spread angle to a decreased desired or offset-corrected spread angle that is less than the increased desired or offset-corrected spread angle;

modify the lost motion compensation function by translating the lost motion compensation function such that the first transition point is shifted to the increased desired or offset-corrected spread angle; and

approximate a lost motion compensation value for the decreased desired or offset-corrected spread angle using the modified lost motion compensation function.

13. The control system of claims 11 or 12, wherein:

the lost motion compensation function comprises:

a first portion that relates desired or offset-corrected spread angle to lost motion compensation value for a range of desired or offset-corrected spread angles; and

a third portion that is representative of a maximum negative lost motion compensation value;

wherein the first portion connects to the third portion at a second transition point, the second transition point being at the smallest desired or offset-corrected spread angle within the range of desired or offset-corrected spread angles; and

the control system is configured to:

receive a request to decrease the spread angle from a desired or offset-corrected spread angle within the range of desired or offset-corrected spread angles to a decreased desired or offset-corrected spread angle that is less than the smallest desired or offset-corrected spread angle within the range of desired or offset-corrected spread angles;

determine a lost motion compensation value for the decreased desired or offset-corrected spread angle using the third portion of the lost motion compensation function that is representative of a maximum negative lost motion compensation value;

subsequently, receive a request to increase the spread angle from the decreased desired or offset-corrected spread angle to an increased desired or offset-corrected spread angle that is greater than the decreased desired or offset-corrected spread angle;

modify the lost motion compensation function by translating the lost motion compensation function such that the second transition point is shifted to the decreased desired or offset-corrected spread angle; and

approximate a lost motion compensation value for the increased desired or offset-corrected spread angle using the modified lost motion compensation function.

14. The control system of any of claims 11 to 13, the control system being further configured to:

determine a commanded spread angle in dependence on the desired spread angle, the offset value and the lost motion compensation value, optionally wherein determining the commanded spread angle in dependence on the desired spread angle, the offset value and the lost motion compensation value comprises:

determining an offset-corrected spread angle in dependence on the desired spread angle and the offset value, approximating a lost motion compensation value in dependence on the offset-corrected spread angle and the lost motion compensation function, and determining the commanded spread angle in dependence on the offset-corrected spread angle and the lost motion compensation value; or

approximating a lost motion compensation value in dependence on the desired spread angle and the lost motion compensation function, and determining the commanded spread angle in dependence on the desired spread angle, the offset value and the lost motion compensation value; and

control the surgical robot arm to drive the robotic surgical instrument using the commanded spread angle.

15. A surgical robotic system comprising:

a surgical robot arm having a drive mechanism configured for driving a robotic surgical instrument, said robotic surgical instrument comprising a first end effector element and a second end effector element, the drive mechanism comprising one or more interface elements configured to interface with the robotic surgical instrument for controlling a spread angle between the first end effector element and the second end effector element; and

a control system according to any of claims 1 to 14.

**FIG. 1**

**FIG. 2**

FIG. 3

FIG. 4

*FIG. 5*

600

618
628
638

632

612
616
626
636

650

622

FIG. 6A

206

101

201

600

314-1

208

310

FIG. 6B

FIG. 7

FIG. 8

902 — Cause an instrument drive force applied by the one or more interface elements to be varied

904 — Predict a resultant spread angle in dependence on a measured position of each of the one or more interface elements

906 — Fit a linear relationship to the at least two applied instrument drive force and predicted resultant spread angle data pairs

908 — Determine a spread offset value

910 — Control the surgical robot arm to drive the robotic surgical instrument in dependence on the spread offset value

## FIG. 9

1002 — Cause an instrument drive force applied by the interface element to be varied

1004 — Measure a position of the interface element which is indicative of a predicted spread angle

1006 — Fit a linear relationship to the at least two applied instrument drive force and measured position data pairs

1008 — Determine an interface position offset value

1010 — Control the surgical robot arm to drive the robotic surgical instrument in dependence on the interface position offset value

## FIG. 10

FIG. 11

FIG. 12

1302 — Cause an instrument drive force applied by the one or more interface elements to be varied

↓

1304 — Predict a resultant spread angle in dependence on a measured position of each of the one or more interface elements

↓

1306 — Determine an instrument stiffness value for each of the plurality of predicted resultant spread angles

↓

1308 — Determine a spread offset value

↓

1310 — Control the surgical robot arm to drive the robotic surgical instrument in dependence on the spread offset value

## FIG. 13

1402 — Cause an instrument drive force applied by the interface element to be varied

↓

1404 — Measure a position of the interface element which is indicative of a predicted spread angle

↓

1406 — Determine an instrument stiffness value for each of the plurality of measured positions

↓

1408 — Determine an interface position offset value

↓

1410 — Control the surgical robot arm to drive the robotic surgical instrument in dependence on the interface position offset value

## FIG. 14

FIG. 15A

FIG. 15B

| | |
|---|---|
| 1602 | Determine one or more interface stiffness values |

$\downarrow$

| | |
|---|---|
| 1604 | Determine a lost motion compensating position of that interface element |

$\downarrow$

| | |
|---|---|
| 1606 | Predict a lost motion compensating resultant spread angle in dependence on the determined lost motion compensating positions of each of the one or more interface elements |

## FIG. 16

| | |
|---|---|
| 1702 | Vary applied instrument drive force |

| | |
|---|---|
| 1704 | Predict a lost motion compensating resultant spread angle |
| 1706 | Determine a spread offset value |

| | |
|---|---|
| 1708 | Determine a calibrated spread angle |

## FIG. 17

*FIG. 18*

1902 — Cause an instrument drive force applied at the one or more interface elements to be varied

1904 — Measure the instrument drive force applied at the one or more interface elements at each of a plurality of times

1906 — Determine a force behaviour value for each of the plurality of times

1908 — Determine an offset value

1910 — Control the surgical robot arm in dependence on the offset value

## FIG. 19

2002 — Receive a request to drive the spread angle to a desired spread angle

2004 — Approximate a lost motion compensation value in dependence on a lost motion compensation function

2006 — Control the surgical robot arm in dependence on the desired spread angle and the lost motion compensation value

## FIG. 20

FIG. 21

FIG. 22A

FIG. 22B

FIG. 23

Europäisches
Patentamt

European
Patent Office

Office européen
des brevets

**EUROPEAN SEARCH REPORT**

Application Number

EP 25 16 5931

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/114494 A1 (NIXON TOM [US]) 15 May 2008 (2008-05-15) | 1-10,15 | INV. A61B34/30 |
| Y | * paragraphs [0064] - [0069]; figures 10-13 * | 11 | ADD. A61B90/00 |
| Y | WO 2022/264075 A2 (MEDICAL MICROINSTRUMENTS SPA [IT]) 22 December 2022 (2022-12-22) | 11 | |
| A | * page 7, line 15 - line 16 * * page 19, line 35 - page 20, line 7 * | 12-14 | |
| X,P | WO 2024/157149 A1 (COVIDIEN LP [US]) 2 August 2024 (2024-08-02) * paragraphs [0091] - [0104]; figures 11-14 * | 1-3,5-9, 15 | |
| X,P | WO 2024/069132 A1 (CMR SURGICAL LTD [GB]) 4 April 2024 (2024-04-04) * the whole document * | 1-15 | |

TECHNICAL FIELDS
SEARCHED      (IPC)

A61B

The present search report has been drawn up for all claims

| Place of search | Date of completion of the search | Examiner |
|---|---|---|
| The Hague | 26 August 2025 | Mayer-Martenson, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 5931

This annex lists the patent family members relating to the patent documents cited in the above-mentioned European search report.
The members are as contained in the European Patent Office EDP file on
The European Patent Office is in no way liable for these particulars which are merely given for the purpose of information.

26-08-2025

| Patent document cited in search report | | Publication date | Patent family member(s) | | Publication date |
|---|---|---|---|---|---|
| US 2008114494 | A1 | 15-05-2008 | US | 2005251110 A1 | 10-11-2005 |
| | | | US | 2008114494 A1 | 15-05-2008 |
| | | | US | 2012083801 A1 | 05-04-2012 |
| | | | US | 2013274922 A1 | 17-10-2013 |
| | | | US | 2014330434 A1 | 06-11-2014 |
| | | | US | 2015314451 A1 | 05-11-2015 |
| | | | US | 2017181805 A1 | 29-06-2017 |
| | | | US | 2018185109 A1 | 05-07-2018 |
| WO 2022264075 | A2 | 22-12-2022 | AU | 2022292193 A1 | 21-12-2023 |
| | | | BR | 112023026623 A2 | 05-03-2024 |
| | | | CA | 3221147 A1 | 22-12-2022 |
| | | | CN | 117940085 A | 26-04-2024 |
| | | | EP | 4355242 A2 | 24-04-2024 |
| | | | JP | 2024523337 A | 28-06-2024 |
| | | | KR | 20240046160 A | 08-04-2024 |
| | | | US | 2024277432 A1 | 22-08-2024 |
| | | | WO | 2022264075 A2 | 22-12-2022 |
| WO 2024157149 | A1 | 02-08-2024 | NONE | | |
| WO 2024069132 | A1 | 04-04-2024 | US | 2024238980 A1 | 18-07-2024 |
| | | | WO | 2024069132 A1 | 04-04-2024 |

EPO FORM P0459

For more details about this annex : see Official Journal of the European Patent Office, No. 12/82